# EUROPEAN PATENT SPECIFICATION

(11) **EP 2 864 493 B1**
(45) Date of publication and mention of the grant of the patent: **17.06.2020**
(21) Application number: 13729968.1
(22) Date of filing: 20.06.2013
(51) Int. Cl.: C12P 21/00

(54) **PRODUCTION OF ENZYMES FOR LIGNO-CELLULOSIC BIOMASS**
HERSTELLUNG VON ENZYMEN FÜR LIGNOZELLULOSEHALTIGE BIOMASSE
PRODUCTION D'ENZYMES POUR BIOMASSE LIGNOCELLULOSIQUE

(30) Priority: 21.06.2012 IT TO20120545
(43) Date of publication of application: 29.04.2015
(73) Proprietor: versalis S.p.A., 20097 San Donato Milanese (MI) (IT)
(72) Inventor: PARAVISI, Stefano, 15057 Tortona (Alessandria) (IT); VOLPATI, Laura, 15057 Tortona (Alessandria) (IT); GIORCELLI, Chiara, I-15027 Pontestura (Alessandria) (IT); RACCAGNI, Elisa, I-15121 Alessandria (IT); OTTONELLO, Piero, I-20143 Milano (IT); CHERCHI, Francesco, I-15067 Novi Ligure (Alessandria) (IT)
(74) Representative: Bottero, Carlo
(86) International application number: PCT/EP2013/062936
(87) International publication number: WO 2013/190064

(56) References cited:
- WO-A1-2011/079388
- WO-A1-2013/053924
- US-A1- 2006 177 917
- KOURTZ ET AL: "High throughput production and characterization of cellulytic and hemicellulytic enzymes", Sixth Annual DOE Joint Genome Institute User Meeting, Walnut Creek, California, 2011, page 33, XP002695526, Retrieved from the Internet: URL:http://www.jgi.doe.gov/meetings/userme eting/2011/JGI-UM6-abstracts.pdf [retrieved on 2013-04-16]
- MTUI: "Lignocellulolytic enzymes from tropical fungi: Types, substrates and applications", SCIENTIFIC RESEARCH AND ESSAYS, vol. 7, April 2012 (2012-04), pages 1544-1555, XP002695527,
- JUHÁSZ ET AL: "Characterization of cellulases and hemicellulases produced by Trichoderma reesei on various carbon sources", PROCESS BIOCHEMISTRY, vol. 38, 2005, pages 3519-3525, XP027794098, cited in the application
- JÖRGENSEN ET AL: "Production of cellulases by Penicillium brasilianum IBT 20888 - Effect of substrate on hydrolytic performance", ENZYME AND MICROBIAL TECHNOLOGY, vol. 38, 2006, pages 381-390, XP027948637, cited in the application
- LIU ET AL: "Screening, identifying of cellulose-decomposing strain L-06 and its enzyme-producing conditions", CHINESE JOURNAL OF BIOTECHNOLOGY, vol. 24, 2008, pages 1112-1116, XP022939166,
- CHIARAMONTI ET AL: "Review of pretreatment processes for lignocellulosic ethanol production, and development od an innovative method", BIOMASS AND BIOENERGY, vol. 46, 23 May 2012 (2012-05-23), pages 25-35, XP055126049,
- ZHANG ET AL: "Effects of acid and alkali treated lignocellulosic materials on cellulase/xylanase production by Trichoderma reesei Rut C-30 and corresponding enzymatic hydrolysis", BIOMASS AND BIOENERGY, vol. 37, 14 January 2012 (2012-01-14), pages 16-24, XP028887699,

## Description

### BACKGROUND

Enzymes are proteins that catalyze biochemical reactions. Enzymes are produced by almost all living host cells. Some enzymes produced by microorganisms of the classes of fungi are useful in the conversion processes of ligno-cellulosic feedstock to chemicals. In these conversion processes, enzymes are used as catalyst in hydrolysis reaction of complex sugars, such as cellulose and hemicellulose, into sugars with lower molecular weight.

It is known that the hydrolysis of cellulose and hemicellulose from ligno-cellulosic feedstocks requires a well-balanced mixture of enzymes consisting of endoglucanases, cellobiohydrolases, β-glucosidases, xylanases, mannanases and various enzymes acting on side chains of xylans and mannans. Enzyme production is an important step in the biomass-to-ethanol process as enzyme or enzyme mixture production and application are currently among the more costly processing steps for biologically based routes to ligno-cellulosic biomass utilization.

The enzyme systems of the plant degrading fungus Trichoderma reesei are the most extensively investigated and believed to be the most widely organism used to obtain commercial enzyme mixtures. T. reesei produces numerous cellulose- and hemicellulose-degrading enzymes even if extracellular β-glucosidase secretion is low. As it is well known that β-glucosidase activity content is critical in order to obtain high cellulose conversion, T. reesei enzyme solution is commonly supplemented with β-glucosidases to obtain a well-balanced enzyme solution and further advance the hydrolysis of the cellulose. In other cases commercial enzyme or enzyme mixture solutions could also be obtained using enzymes produced by other, good β-glucosidase-producing fungi.

The enzymes for conversion processes of ligno-cellulosic feedstocks are produced by cultivating the host cells on complex media comprising pure cellulose and/or even more expensive carbon sources.

For reducing the final cost of enzyme production, many attempts have been done for using substrates less expensive than pure cellulose. Pretreated ligno-cellulosic feedstocks have been used as a substrate. Many research groups had tried to produce enzyme or enzyme mixtures using in some manner the same feedstock used for bio-ethanol production to reduce final enzyme mixture costs.

However, no clear relationship between the substrate used for cultivation and the hydrolytic performance of the resulting enzymes on the particular substrates has been reported in previous studies on T. reesei Rut C30 or Penicillium brasilianum. A review of results obtained in the cultivation of enzymes on different carbon sources can be found in Juhasz, T., Szengyel, Z., Reczey, K., Siika-Aho, M., Viikari, L. "Characterization of enzyme or enzyme mixtures and hemienzyme or enzyme mixtures produced by Trichoderma reesei on various carbon sources", (Process Biochem, 40, 3519-3525) and in Jorgensen, H., Olsson, L., "Production of cellulases by Penicillium brasilianum IBT 20888: Effect of substrate on hydrolytic performance" (Enzyme Microb Technol, 38, 381-390). One such process is described in WO 2007/005918. This process adds the described pre-treated ligno-cellulosic substrate as an inducer of enzyme growth, while using constant addition of glucose as the feed for the organism growth. The stated purpose of this technology is to replace glucose feed and pure cellulose used today for enzyme or enzyme mixture production in a host cell, with a material that could be used either as the main carbon source and as the inducer for enzyme production. The advantage of this is that the production cost is reduced due to use of an inducer (ligno-cellulosic biomass) which is easily available and thus cheaper than pure cellulose. As an inducer, WO 2007/005918 uses a small amount of biomass and continually adds glucose to feed the organism.

US 7,494,792 discloses a process for producing cellulolytic and/or hemicellulolytic enzymes which uses the residue from the ethanolic fermentation of enzymatic hydrolyzates of cellulosic or ligno-cellulosic biomass. The process may be integrated into a process for the production of ethanol from cellulosic or ligno-cellulosic biomass, in which the residue serves for the production of the enzymes used in enzymatic hydrolysis of the pre-treated substrate. The sugar solution obtained for ethanol production from hydrolysis is separated from the non hydrolyzed solid fraction, essentially constituted by lignin and it is used for ethanolic fermentation. Glucose is the major sugar withdrawn at this step. The residue from ethanolic fermentation, after separating the ethanol, is used as a source of inducing carbon or as a principal source of carbon for the production of enzymes.

As a general consideration, there is the interest in converting all or the most relevant fraction of the pretreated ligno-cellulosic feedstock into valuable products. The use of a fraction of pretreated ligno-cellulosic feedstock as a substrate for enzyme production may reduce the yield of a plant for the conversion process of ligno-cellulosic feedstock to useful products.

A review of the main options available in biomass pretreatment has been found in Chiaramonti D., Prussi M., Ferrero S., Oriani L., Ottonello P., Torre P. and Cherchi F. "Review of pretreatment processes for lignocellulosic ethanol production, and development of an innovative method" (Biomass and Bioenergy (2012) 46, 25-35). In particular, this work concerns a new process using only water and steam as reacting media instead of autohydrolysis and steam explosion processes. As regards the effect of pretreatment on enzymatic hydrolysis, said new pretreatment process achieved yields similar to steam explosion on glucans: however, this was obtained reducing the formation of degradation products from sugars, mainly from C5 sugars. These results made the proposed pretreatment system suitable for further development and industrialization on pilot and industrial scale. Therefore, there is the need to develop a process which better uses a ligno-cellulosic pretreated feedstock for producing enzymes.

### SUMMARY

The invention refers to a process as defined in the claims, namely a process to produce at least a first enzyme capable of hydrolysing a first pre-treated ligno-cellulosic biomass, comprising the step of cultivating the host cell in a sugar-depleted cultivation environment comprising the host cell, water and a solid composition, obtained from a second pre-treated ligno-cellulosic biomass comprising a complex sugar selected from the group of polymeric water-insoluble glucans and xylans, and a lignin; wherein the ratio of the total amount of the complex sugars (glucans and xylans) to the total amount of the lignin in the solid composition is less than the ratio of the total amount of the complex sugars to the total amount of the lignin in the second pre-treated ligno-cellulosic biomass.

Preferred embodiments of the method of the invention are as defined in the dependent claims. It is also disclosed that the cultivation may be done under simple sugar-depleted conditions of having an amount of added simple sugar or sugars in the range of 0 to 10% by weight of the sugar-depleted cultivation environment on a dry basis for a portion of the cultivation time which is at least 50% of the cultivation time.

It is further disclosed that the solid composition may be obtained from the second pre-treated ligno-cellulosic biomass by a process comprising the steps of hydrolysing at least a fraction of the second pre-treated ligno-cellulosic biomass in the presence of a hydrolysis catalyst, to produce a hydrolysed composition comprising the solid composition and a solution of water and water-soluble sugars; and removing at least 50% of the solution of water and water-soluble sugars from the hydrolyzed composition.

It is also disclosed that the removal of a fraction of the hydrolyzed composition may be done by at least one method comprised in the group of decantation, sedimentation, centrifugation, filtration, press filtration and washing.

It is further disclosed that the solid composition may be obtained from the second pre-treated ligno-cellulosic biomass by a process comprising the steps of hydrolysing at least a fraction of the second pre-treated ligno-cellulosic biomass in the presence of a hydrolysis catalyst, to produce a hydrolysed composition comprising water-soluble sugars complex sugars and lignin; converting at least a fraction of the water-soluble sugars in the hydrolysed composition to produce a converted composition comprising at least one sugar-derived product, and the solid composition; and removing a fraction of the converted composition, said fraction comprising at least a fraction of the at least one sugar-derived product.

It is also disclosed that the removal of a fraction of the converted composition may be done by at least one method comprised in the group of decantation, sedimentation, centrifugation, filtration, press filtration, washing, evaporation and distillation.

It is further disclosed that the hydrolysis catalyst may comprise a second enzyme or enzymes mixture, and that it may be selected from the group consisting of hydrogen ions, organic acids and inorganic acids.

It is also disclosed that the conversion of the hydrolysed composition may comprise a fermentation.

It is further disclosed that the at least one sugar-derived product may comprise ethanol.

It is further disclosed that the portion of the cultivation time under simple sugar-depleted conditions may be selected from the group consisting of at least 75% of the cultivation time, at least 85% of the cultivation time, at least 90% of the cultivation time, at least 95% of the cultivation time and at least 98% of the cultivation time.

It is also disclosed the portion of the cultivation time under simple sugar-depleted conditions may be the same as the cultivation time.

It is further disclosed that the amount of added simple sugar or sugars is in the range of between 0 to 5%, 0 to 2.5%, 0 to 2.0%, 0 to 1.0% by weight of the cultivation environment on a dry basis. It is also disclosed that there may be no added simple sugar in the sugar-depleted cultivation environment.

It is further disclosed that at least the first enzyme is harvested by separating the sugar-depleted cultivation environment into at least a harvested composition, comprising at least a fraction of the first enzyme, and an exhausted composition, comprising at least a fraction of the solid residue of the solid composition.

It is also disclosed that the enzyme or enzyme mixture may be further used to hydrolyze the first ligno-cellulosic biomass and the first ligno-cellulosic biomass and the second pretreated ligno-cellulosic biomass both comprise ligno-cellulosic biomass derived from group consisting of the same grass genus or more preferably the same grass species.

It is further disclosed that the total amount of complex sugars in the exhausted composition may be present in a range selected from the group consisting of 0 to 30%, 0 to 20%, 0 to 15%, 0 to 10% , 0 to 7.5% , 0 to 5% , 0 to 2.5% by weight on a dry basis.

### Brief Description of Figures

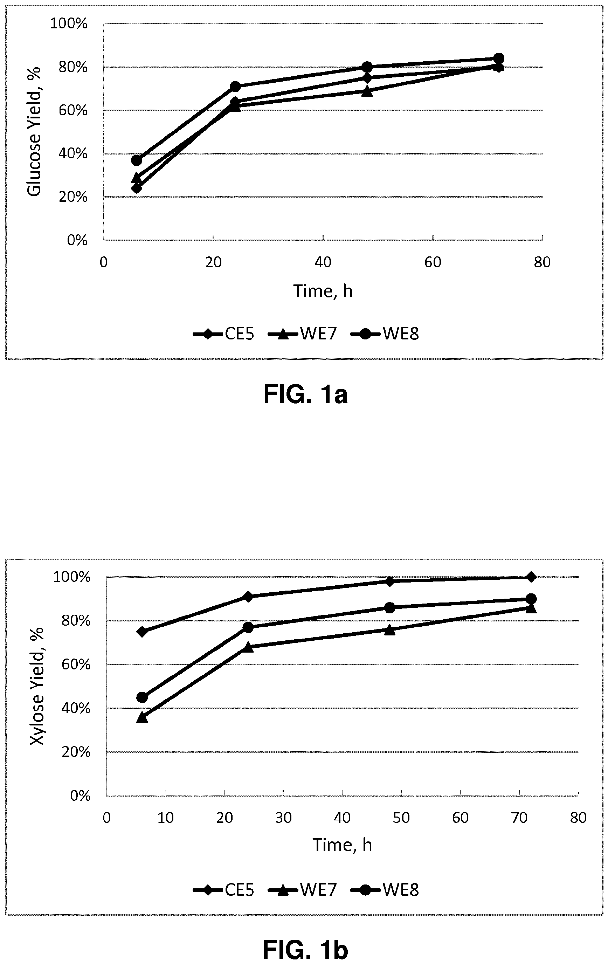

Figure 1a is a graph of the glucose hydrolysis yield according to comparative and working examples.
Figure 1b is a graph of the xylose hydrolysis yield according to comparative and working examples.

### Detailed Description

It has been discovered a process for the production of enzymes, characterized by the fact that host cells which produce the enzymes are cultivated in a sugar depleted cultivation environment. The sugar-depleted cultivation environment comprises the host cells, water and a solid composition which is obtained from a pre-treated ligno-cellulosic biomass; the solid composition comprises complex sugars and lignin, and it is characterized by a ratio of the total amount of the complex sugars to the total amount of the lignin less than the ratio of the total amount of the complex sugars to the total amount of the lignin of the pre-treated ligno-cellulosic biomass from which it is derived. In other words, the solid composition in the sugar-depleted cultivation environment is obtained from a pre-treated ligno-cellulosic biomass by removing a fraction, but not all, of the complex sugars contained in the pre-treated ligno-cellulosic biomass. The solid composition in the sugar-depleted cultivation environment has a lower amount of complex sugars on a dry basis with respect to the pre-treated ligno-cellulosic biomass, but still comprises complex sugars.

The sugars that have been removed from the pre-treated ligno-cellulosic biomass may be converted to useful chemicals, such as ethanol.

The enzymes produced by the host cells may be used then in the hydrolysis of ligno-cellulosic biomass and pre-treated ligno-cellulosic biomass. Cellulose, hemicellulose and lignin are the three main polymers that constitute ligno-cellulosic biomass. Cellulose and hemicellulose are polymeric sugars.

In the context of the present disclosure, simple sugars are the monomeric sugars, and may be selected from the group consisting of glucose, xylose, arabinose, mannose, galactose, and fructose. It should be noted that there may be other simple sugars not in the preceding list. Simple sugars belong to the group of water-soluble sugars. Soluble sugars are sugars, which when in the presence of a specified solvent, such as water, form a homogeneous solution in the solvent.

Simple sugars may feed the host cell directly, that is the host cell does not need to use enzymes to metabolize simple sugars.

Oligomeric sugars are polymeric sugars that are water-soluble. Oligomeric sugars are constituted by more than one monomeric sugar. Oligomeric sugars can be metabolized by the host cell only in the presence of one or more enzymes, which is usually produced by the host cell.

Complex sugars are polymeric sugars that are not soluble in water, such as water-insoluble glucans and xylans, as defined in claim 1. Glucans are a class of complex sugars which comprise polymer molecules of glucose having different degrees of polymerization. Xylans are a class of complex sugars which comprise polymer molecules of xylose having different degrees of polymerization.

Many conversion processes of ligno-cellulosic biomass into chemical products convert only a fraction of complex sugars, and produce a composition containing mainly lignin and the remaining fraction of unconverted complex sugars as a by-product. In some cases, even if it is possible to convert further the complex sugars in the pretreated ligno-cellulosic biomass, the conversion process is stopped because it would be economically not convenient. The composition still containing the remaining fraction of complex sugars is often burnt for producing heat, that is a low value application of sugars contained in ligno-cellulosic biomass.

The inventors have surprisingly found that host cells may be cultivated in a sugar-depleted cultivation environment comprising a solid composition obtained by removing at least a fraction of complex sugars from a pretreated ligno-cellulosic biomass and that the host cells can use the remaining fraction of complex sugars as a carbon source. The inventors have also found that the production of enzymes from the host cell may be comparable or increased with respect to the production of enzyme obtained by using a more expensive substrate as a carbon source.

The simple sugars such as glucose and xylose used to traditionally feed host cells may be replaced partly or entirely by the less expensive solid composition.

Preferably, the sugar-depleted cultivation environment contains no, or few, simple sugars.

Any method or combination of methods known in the art and still to be invented may be used for obtaining the solid composition from a second pre-treated ligno-cellulosic biomass. The methods may involve the use of chemical, physical, biological processes or combination of processes.

Preferably, complex sugars are removed from the pretreated ligno-cellulosic biomass and converted to sugar-derived products, which are valuable chemical products. The conversion process may be realized in a single step or in multiple steps, that is the sugar-derived products may be obtained by converting removed complex sugars to intermediate products.

In a preferred embodiment, the solid composition may be obtained by hydrolyzing at least a fraction of the complex sugars in the pretreated ligno-cellulosic biomass.

Hydrolysis is a process which converts complex sugars into watersoluble sugars and oligomeric sugars into sugars having lower molecular weight.

The hydrolysis of the pre-treated biomass is conducted in the presence of a hydrolysis catalyst and eventually other additives, which may be helpful or necessary for the hydrolysis effectively occurring. In the context of the present invention, the hydrolysis catalyst and the additives may be referred to as a "hydrolysis catalyst composition".

In a preferred embodiment, the hydrolysis catalyst composition comprises an enzyme or enzyme mixture and the hydrolysis is an enzymatic hydrolysis.

In another preferred embodiment, the enzymatic hydrolysis is conducted according to the process disclosed in WO 2010/113130.

In another embodiment, the hydrolysis catalyst may comprise an acid and the hydrolysis is an acid hydrolysis. All the organic and inorganic acids able to perform a hydrolysis of the second pre-treated ligno-cellulosic biomass may be used. Acid may also be present in diluted solutions. Hydrogen ions may also be used in the hydrolysis process.

The hydrolysis of the pretreated ligno-cellulosic biomass may occur in a liquid environment. The liquid environment may be obtained by adding some liquid, preferably water or comprising water, to the pretreated ligno-cellulosic biomass. Liquids may also be added in the pre-treatment of the ligno-cellulosic biomass. For instance, in a preferred embodiment the ligno-cellulosic biomass is pretreated by soaking the ligno-cellulosic biomass in a liquid comprising water, and then steam exploding the soaked ligno-cellulosic biomass. Liquid may also be added in the hydrolysis step or to the hydrolysis catalyst composition. In the case of enzymatic hydrolysis, liquid may be present in the enzymatic mixture. In the case of acid hydrolysis, liquid may be present in the diluted acid solution.

As the hydrolysis of the pretreated ligno-cellulosic biomass occurs in a liquid environment, preferably comprising water, the hydrolyzed composition obtained from the ligno-cellulosic biomass will comprise a solution of water and water-soluble sugars, and a solid composition, comprising complex sugars which have not been hydrolyzed into water-soluble sugars. The solid composition of the hydrolyzed composition comprises also the lignin contained in the pretreated ligno-cellulosic biomass. The lignin may also have been modified and/or partly converted into other components as an effect of the pre-treatment and hydrolysis processes.

Even if the hydrolyzed composition may include other components, for the scope of the present invention, the hydrolyzed composition comprises at least a solution of water and water-soluble sugars and a solid composition further comprising complex sugars and lignin of the solid composition.

The solid composition may be obtained by removing at least 50% of the solution of water and water-soluble sugars from the hydrolyzed composition. It is noted that, while removing the largest amount of the solution of water and water-soluble sugars as possible is a preferred embodiment, some water-soluble sugars may still be present in the solid composition. In another embodiment, the solid composition does not comprise water-soluble sugars.

The fraction removed from the hydrolyzed composition, comprising water and water-soluble sugars, may further comprise other components of the hydrolyzed composition, such as at least a fraction of the hydrolysis catalyst and additives or compounds which are formed during the pre-treatment or the hydrolysis. The fraction removed may further comprise a fraction of the solid composition comprising lignin and complex sugars. In a preferred embodiment, the sugar-depleted environment comprises substantially all the solid composition in the hydrolyzed composition and substantially all the solution of water and water-soluble sugars is removed.

In another embodiment, the sugar-depleted environment comprises all the solid composition in the hydrolyzed composition and all the solution of water and simple sugars is removed.

Any methods and combination of methods know in the art and even still to be invented may be used for removing at least 50% of solution of water and water-soluble sugars from the hydrolyzed composition. These methods comprise chemical and physical treatments of the hydrolysed composition. The chemical and physical treatments may be carried out sequentially or simultaneously.

Preferred methods for removing at least a fraction of the hydrolysed composition comprise decantation, sedimentation, centrifugation, filtration, including membrane filtration and press filtration and washing.

The hydrolysis of at least a fraction of the pre-treated ligno-cellulosic biomass and the removal of at least 50% of the solution of water and water-soluble sugars may occur sequentially or at least partially simultaneously. In one embodiment, at least a fraction of the simple sugars may be removed, for instance by membrane filtration, while hydrolysis is proceeding.

The solid composition may be further processed prior to being inserted in the sugar-depleted cultivation environment of the host cells for the production of enzymes. The solid composition may also be washed, preferably with water, for further removing watersoluble sugars or other components that may affect or reduce the production of the enzymes. Other components needed for cultivating the host cells may be added to the solid composition or to the sugar-depleted cultivation environment.

The fraction of the solution of water and water-soluble sugars removed from the hydrolyzed composition, may be converted to useful chemical products.

In another preferred embodiment, the solid composition may be obtained by hydrolyzing at least a fraction of the complex sugars in the pretreated ligno-cellulosic biomass in the presence of a hydrolysis catalyst to produce a hydrolysed composition comprising complex sugars and lignin and the solution of water and water-soluble sugars; then, converting at least a fraction of the water-soluble sugars in the hydrolysed composition in a converted composition comprising at least one sugar-derived product and a solid composition comprising complex sugars and lignin; and removing at least a fraction of the sugar-derived product from the converted composition.

All the considerations on hydrolysis previously stated in the present specification apply also for this embodiment. The hydrolysed composition may be subjected to further processing within the scope of the invention. For instance, the process may improve the conversion of the water-soluble sugars into products and may include physical and chemical treatments, for example, separation, purification, pH correction, dilution, and concentration. Water-soluble sugars in the hydrolyzed composition are converted, partly or entirely, to a converted composition, which comprises at least one sugar-derived product.

Conversion or modification of any other component of the hydrolyzed composition may also occur during the conversion of the simple sugars. In particular, the complex sugars and lignin in the hydrolyzed composition may be affected by the conversion process to generate a solid composition comprising complex sugars and lignin; stated in another way, the solid composition may be the complex sugars and lignin in the hydrolyzed composition or a modification of complex sugars and lignin in the hydrolyzed composition. The converted composition may further comprise more than one product derived from water-soluble sugars.

The conversion of the water-soluble sugars may occur in the presence of a catalyst. The catalyst may eventually comprise other additives, which may be helpful or necessary for the

conversion effectively occurring. In the context of the present invention, the catalyst and the additives may be referred to as a "catalyst composition".

The catalyst may be any agent which promotes the conversion of water-soluble sugars to at least one sugar-derived product. The catalyst may be an inorganic, organic or biological agent.

Even if any process may be used for converting at least a fraction of the hydrolysed composition, fermentation is a preferred process and the sugar-derived product is a fermented product. Fermentation is any process to convert simple sugars into fermented products in the presence of a microorganism or microorganism mixture.

A preferred microorganism for converting at least a fraction of the hydrolysed composition is a yeast.

Yeast commonly indicates an unicellular chiefly ascomycetous fungus that has usually little or no mycelium, that typically reproduces asexually by budding with the daughter cells often remaining attached and that are capable of fermenting carbohydrates into alcohol and carbon dioxide. Preferably, the yeast belongs to the genus Saccharomyces. Species of Saccharomyces are as defined phylogenetically by Kurtzman (2003) FEMS Yeast Research 3:417-432, and include S. cerevisiae, S. paradoxus, S. ikatae, S. cariocanus, S. kudriavzevii , S. pastorianus and S. bayanus.

Depending on the catalyst or microorganism, different sugar-derived products may be produced. Even if any other sugar-derived products may be produced, in a preferred embodiment the sugar-derived product is ethanol. Hydrolysis of complex sugars to a hydrolyzed composition and conversion of at least a fraction of the water-soluble sugars in the hydrolyzed composition to at least one sugar-derived product may occur sequentially or simultaneously. In a preferred embodiment, enzymatic hydrolysis and fermentation to ethanol occur simultaneously and the process is known in the art as Simultaneous Saccharification and Fermentation.

The conversion of at least a fraction of the water-soluble sugars in the hydrolyzed composition may occur in a liquid environment. The liquid environment may be obtained by adding some liquid, preferably water or comprising water, to the hydrolyzed composition. Liquids may also be present in the hydrolyzed composition or in the catalyst composition.

As the conversion of at least a fraction of the water-soluble sugars occurs in a liquid environment, preferably comprising water, the converted composition will usually comprise the at least one sugar-derived product, water, and a solid composition, comprising complex sugars and lignin. The lignin and complex sugars in the hydrolyzed composition may also be modified and/or partly converted into other components as an effect of the hydrolysis and conversion process. Water-soluble sugars which have not been converted into products may also be comprised in the converted composition.

For the scope of the present invention, the converted composition comprises at least one sugar-derived product and a solid composition comprising complex sugars and lignin. The at least one sugar-derived product may be soluble or insoluble in the converted composition. In a preferred embodiment, the sugar-derived product is soluble in the converted composition. For instance, ethanol is soluble in the fermented composition. The solid composition is obtained by removing a fraction of the converted composition comprising at least a fraction of the sugar-derived product. It is noted that, while removing the largest amount of the sugar-derived product as possible is a preferred embodiment, some sugar-derived product may still be present in the solid composition. In another embodiment, solid composition does not comprise sugar-derived product(s).

The fraction removed from the converted composition may further comprise other components of the converted composition, such as water-soluble sugars which have not been converted, water, at least a fraction of the catalyst and additives. It may further comprise a fraction of the solid composition comprising lignin and complex sugars of the converted composition. In a preferred embodiment, the sugar-depleted environment comprises substantially all the solid composition in the converted composition and the fraction removed from the converted composition comprises substantially all the liquids in the converted composition.

In another embodiment, the sugar-depleted environment comprises all the solid composition in the converted composition and the fraction removed from the converted composition comprises all the liquids in the converted composition.

Any methods and combination of methods know in the art and even still to be invented may be used for removing a fraction of converted composition, comprising the at least one sugar-derived product. These methods comprise chemical and physical treatments of the converted composition. The chemical and physical treatments may be carried out sequentially or simultaneously.

Preferred methods for removing at least a fraction of the converted composition comprise decantation, sedimentation, centrifugation, filtration, including membrane filtration and press filtration, washing, evaporation and distillation.

In a preferred embodiment, the converted product is ethanol and the solid composition is obtained from the converted composition by evaporating or distilling ethanol and removing the most fraction of the liquids in the converted composition by at least a method in the group of decantation, sedimentation, centrifugation, filtration, including membrane filtration and press filtration and washing.

The conversion of the water-soluble sugars in the hydrolyzed composition to at least one sugar-derived product and the removal of a fraction of the converted composition comprising at least a fraction of the converted product may occur at least partially simultaneously. In an embodiment, the converted product is ethanol and it may be removed, for instance by evaporation or distillation, while simple sugars in the hydrolyzed composition are fermented to ethanol.

In another embodiment, hydrolysis of the pre-treated ligno-cellulosic biomass, conversion of at least a fraction of the water-soluble sugars and removal of a fraction of the converted composition occur at least partially simultaneously. In a preferred embodiment, the converted product is ethanol and it may be removed, for instance by evaporation and distillation, during Simultaneous Saccharification and Fermentation.

The solid composition may be further processed prior to be inserted in the sugar-depleted cultivation environment of the host cells for the production of enzymes. The solid composition may also be washed, preferably with water, for removing further watersoluble sugars or other components that may affect or reduce the production of the enzymes.

### Process of Producing Enzymes

It is well known in the art how to produce enzyme or enzyme mixture in a host cell of fungal origin, such as filamentous fungi, or bacteria origin. The growth process of the invention may be a well-known process, except that the feed comprises a solid composition obtained from a pre-treated ligno-cellulosic biomass and having a total amount of complex sugars lower than the total amount of complex sugars in the pre-treated ligno-cellulosic biomass. In a preferred embodiment, the feed of simple sugars is limited during cultivation.

Enzyme production procedures are well known in the art. In context of the present invention the enzyme or enzyme mixture is preferably an extra-cellular enzyme or enzyme mixture secreted into the cultivation environment by the host cell. Alternatively, the enzyme or enzyme mixture is intracellular.

A host cell capable of producing enzyme or enzyme mixture is grown under precise cultural conditions at a particular growth rate. When the host cell culture is introduced into the cultivation environment the inoculated culture passes through a number of stages. Initially growth does not occur. This period is referred to as the lag phase and may be considered a period of adaptation. During the next phase referred to as the "exponential phase" the growth rate of the host cell culture gradually increases. After a period of maximum growth the rate ceases and the culture enters stationary phase. After a further period of time the culture enters the death phase and the number of viable cells declines. When, in the growth phase the enzyme, or enzyme mixture of interest is expressed depends on the enzyme of interest and the host cell. The enzyme or enzyme mixture may, in one embodiment, be expressed in the exponential phase. In another embodiment, the enzyme or enzyme mixture may be produced in the transient phase between the exponential phase and the stationary phase. The enzyme or enzyme mixture may also, in another embodiment, be expressed in the stationary phase and/or just before sporulation. The enzyme or enzyme mixture may, according to the invention, also be produced in more than one of the above mentioned phases.

In other words, according to the invention the host cell is cultivated in a suitable environment and under conditions allowing at least one enzyme or enzyme mixture to be expressed, preferably secreted and optionally recovered. While as noted above, the host cell growth has many technical phases, for the purposes of this specification, these phases are grouped together in the term cultivation. Host cell cultivation takes place in a sugar-depleted cultivation environment comprising a solid composition obtained from a pre-treated ligno-cellulosic biomass by removing at least a fraction of complex sugars. The sugar-depleted cultivation environment may further comprise an additional carbon source, a nitrogen source and additional salts required for microorganism metabolism.

According to a preferred embodiment the pre-treated ligno-cellulosic biomass has been pre-treated. by being soaked/washed and then steam exploded as described in WO 2010/113129.

After cultivation the enzyme or enzyme mixture may optionally be recovered using methods well known in the art. For example, extra-cellular enzyme or enzyme mixture recovery from the sugar-depleted cultivation environment may be done using conventional procedures including, but not limited to, centrifugation, filtration, extraction, spray-drying, evaporation, or precipitation. Procedures for recovery of an intracellular enzyme or enzyme mixture are also well known in the art.

At least in context of the present invention the term "cultivation" means any process of producing an enzyme or enzyme mixture using a mass culture consisting of one or more host cells. The present invention is useful for especially industrial scale production, e.g., having a cultivation environment of at least 50 liters, preferably at least 5 liters, more preferably at least 1 liter.

The enzyme or enzyme mixture may include, but is not limited to any of those belonging to the group of enzyme or enzyme mixture comprising endoglucanases (endo-1,4-β-D-glucanase), cellobiohydrolases or exoglucanases (exo-1,4-β-D-glucanase), β-glucosidase (1,4-β-D-glucosidase), endo-1,4-*β*-xylanase, endo-1,4-*β*-mannanase, 1,4-*β*-xylosidase and 1,4-*β*-mannosidase.

A process of the invention may be performed as a batch, a fed-batch, a repeated fed-batch or a continuous process.

A process of the invention may be carried out aerobically or anaerobically. Some enzymes are produced by submerged cultivation and some by surface cultivation. Submerged cultivation is preferred according to the invention.

Thus, according to one aspect, the invention, as defined in the claims, relates to processes of producing an enzyme or enzyme mixture in a host cell comprising cultivating said host cell capable of producing enzyme or enzyme mixture under conditions conducive for production of an enzyme or enzyme mixture, such as enzyme or enzyme mixture, wherein a solid composition obtained from a pre-treated ligno-cellulosic biomass is used to grow the host cell under simple sugar-depleted conditions of having an amount of added simple sugar or sugars in the range of 0 to 10% by weight of the sugar-depleted cultivation environment on a dry basis for a portion of the cultivation time which is at least 50% of the cultivation time. The cultivation time is the amount of time measured from the addition of the host cell preculture volume to the sugar-depleted cultivation environment to the harvest, removal, or separation of the enzyme or enzyme mixture from the sugar-depleted cultivation environment. In the case of multiple removals, the cultivation time ends at the time when the last removal of the first enzyme or enzyme mixture is ended.
The amount of the solid composition obtained from pre-treated ligno-cellulosic biomass present in the sugar-depleted cultivation environment should be sufficient for the growth of the host cell to produce the enzyme or enzyme mixture.

The phrase simple sugar-depleted conditions means generally that more than 50% by weight of the host cell feed is from the solid composition obtained from a second pre-treated ligno-cellulosic biomass and not from added simple sugars. An exemplary simple sugar-depleted condition is when the amount of optional simple sugars added to the process, if any is added at all, is in the range of 0 to 10% by weight of the sugar depleted cultivation environment on a dry basis. More preferably, the optional simple sugars added should be in the range of 0 to 5% by weight of the sugar-depleted cultivation environment on a dry basis, with 0 to 2.5 % by weight being even more preferred, with 0 to 2.0% being the most preferred (if simple sugars are added at all). In the best case, there are no simple sugars added which is the perfect simple sugar-depleted condition. Additionally, the phrase simple sugars added means that there could be one or more simple sugars added. In one embodiment the optional simple sugar is present, but at less than the percentage indicated.

The simple sugar-depleted conditions should be maintained for at least a portion of the cultivation time. Expressed quantitatively, the simple sugar-depleted conditions should be maintained for at least 50% of the cultivation time, with 75% being more preferred, 85% being even more preferred, with 95% being even yet more preferred with 99 and 100% of the cultivation time being the most preferred. 100% of the cultivation time is when the at least a portion of the cultivation time equals the cultivation time.

### The Substrate

Carbon source substrates commonly used as feed for enzyme or enzyme mixture production includes glucose or similar sugars, provided their consumption relative to the consumption of the complex sugars is within the specified boundaries. Nitrogen source substrates, growth stimulators and the like may be added to improve cultivation and enzyme or enzyme mixture production. Nitrogen sources include urea, ammonia salts (for example NH₄Cl or NH₄ SO₄) and peptides. Protease may be used, e.g., to digest proteins to produce free amino nitrogen (FAN). Such free amino acids may function as nutrient for the host cell, thereby enhancing the growth and enzyme or enzyme mixture production. Preferred cultivation stimulators for growth include vitamins and minerals. Examples of vitamins include multivitamins, biotin, pantothenate, nicotinic acid, meso-inositol, thiamine, pyridoxine, para-aminobenzoic acid, folic acid, riboflavin, and Vitamins A, B, C, D, and E. Examples of minerals include minerals and mineral salts that can supply nutrients comprising P, K, Mg, S, Ca, Fe, Zn, Mn, and Cu.

Pure cellulose, usually used as an inducer (and carbon source) in enzyme or enzyme mixture production processes, is replaced with a solid composition obtained from a pre-treated ligno-cellulosic biomass; the composition is preferably detoxified if acid pre-treated, for instance by washing.

The solid composition is a carbon source and may be added to the sugar depleted cultivation environment together with a carbon source, but may also be added separately from the carbon source. According to the invention the solid composition may be added to the sugar-depleted cultivation environment either prior to inoculation, simultaneously with inoculation or after inoculation of the host cell culture in an amount at least corresponding to the amount of available complex sugars needed to grow the host cell. When the solid composition is added during the cultivation time, a new calculation of the amount optional simple sugars added or the ratio of optional simple sugars to ligno-cellulosic biomass is done. While the amount of simple sugars may not have been low enough during the initial part of the cultivation time, by adding the solid composition to the sugar-depleted cultivation environment, the amount of optional simple sugars added would fall within the specified ranges, at least for the time remaining in the cultivation time.

A person skilled in the art can easily determine when to add and what amount of the solid composition according to the method of the invention. During the time span of cultivation the solid composition is normally consumed by the cell host and kept within the previously specified limits.

As mentioned above the solid composition is used the same way glucose is normally used in well-known enzyme or enzyme mixture production processes.

### Enzyme(s) harvesting

In the disclosed process, the solid composition is used for feeding host cells in the enzyme production process. The solid composition is obtained from a pre-treated ligno-cellulosic biomass, said solid composition comprising complex sugars and lignin. As the host cells are fed by the complex sugars in the solid composition, a solid residue of the solid composition is formed from at least a fraction of the solid composition in the sugar-depleted cultivation environment. The solid residue comprises at least a fraction of the lignin of the solid composition and may comprise a fraction of the complex sugars of the solid composition. The lignin may have been modified in the host cells cultivation step.

The enzymes produced by cultivating the host cells in the sugar-depleted cultivation environment may be harvested by separating the sugar-depleted cultivation environment in at least two fractions, the harvested composition, comprising at least a fraction of the enzymes, and the exhausted composition, comprising at least a fraction of the solid residue of the solid composition. The solid residue may comprise most of the host cells.

By "at least two fractions" it is meant that the sugar-depleted cultivation environment may be separated in more than two fractions, this embodiment being within the scope of the invention.

The harvested composition may further comprise other components of the sugar-depleted cultivation environment, such as for instance water and additives contained in the solid composition and/or sugar-depleted cultivation environment and/or added in the host cells cultivation. It may further comprise some of the solid composition and some of the solid residue. In a preferred embodiment, the harvested composition contains no, or very few, solid compounds. In another preferred embodiment, the harvested composition contains all, or almost all, the produced enzymes.

The exhausted composition comprises at least a fraction of the solid residue of the solid composition and may further comprise other components of the sugar-depleted cultivation environment, such as for instance water and additives contained in the sugar-depleted environment and/or added in the host cells cultivation step. It may further comprise some of the enzymes. In a preferred embodiment, the exhausted composition contains no, or very few, enzymes. In another preferred embodiment, the exhausted composition contains all, or almost all, the solid residue of the solid composition.

Any methods and combination of methods know in the art and even still to be invented may be used for separating the sugar-depleted cultivation environment in at least the harvested composition and the exhausted composition. These methods comprise chemical and physical treatments of the hydrolysed composition. The chemical and physical treatments may be carried out sequentially or simultaneously.

Preferred methods for separating the sugar-depleted cultivation environment in at least the harvested composition and the exhausted composition comprise at least a method in the group of centrifugation, filtration, dialysis, washing and press filtration.

The harvested composition and the exhausted composition may be subjected to any further process within the scope of the invention. For instance, for examples separation, purification, pH correction, dilution, concentration.

### The Ligno-cellulosic Biomass

In general, a natural or naturally occurring ligno-cellulosic biomass can be described as follows.

Apart from starch, the three major constituents in plant biomass are cellulose, hemicellulose and lignin, which are commonly referred to by the generic term lignocellulose. Polysaccharide-containing biomasses as a generic term include both starch and ligno-cellulosic biomasses. Therefore, some types of feedstocks can be plant biomass, polysaccharide containing biomass, and ligno-cellulosic biomass.

Polysaccharide-containing biomasses include any material containing polymeric sugars e.g. in the form of starch as well as refined starch, cellulose and hemicellulose.

Relevant types of naturally occurring biomasses for deriving the claimed invention may include biomasses derived from agricultural crops selected from the group consisting of starch containing grains, refined starch; corn stover, bagasse, straw e.g. from rice, wheat, rye, oat, barley, rape, sorghum; softwood e.g. Pinus sylvestris, Pinus radiate; hardwood e.g. Salix spp. Eucalyptus spp.; tubers e.g. beet, potato; cereals from e.g. rice, wheat, rye, oat, barley, rape, sorghum and corn; waste paper, fiber fractions from biogas processing, manure, residues from oil palm processing, municipal solid waste or the like. Although the experiments are limited to a few examples of the enumerated list above, the invention is believed applicable to all because the characterization is primarily to the unique characteristics of the lignin and surface area.

The ligno-cellulosic biomass feedstock used to derive the composition is preferably from the family usually called grasses. The proper name is the family known as Poaceae or Gramineae in the Class Liliopsida (the monocots) of the flowering plants. Plants of this family are usually called grasses, or, to distinguish them from other graminoids, true grasses. Bamboo is also included. There are about 600 genera and some 9,000-10,000 or more species of grasses (Kew Index of World Grass Species).

Poaceae includes the staple food grains and cereal crops grown around the world, lawn and forage grasses, and bamboo. Poaceae generally have hollow stems called culms, which are plugged (solid) at intervals called nodes, the points along the culm at which leaves arise. Grass leaves are usually alternate, distichous (in one plane) or rarely spiral, and parallel-veined. Each leaf is differentiated into a lower sheath which hugs the stem for a distance and a blade with margins usually entire. The leaf blades of many grasses are hardened with silica phytoliths, which helps discourage grazing animals. In some grasses (such as sword grass) this makes the edges of the grass blades sharp enough to cut human skin. A membranous appendage or fringe of hairs, called the ligule, lies at the junction between sheath and blade, preventing water or insects from penetrating into the sheath.

Grass blades grow at the base of the blade and not from elongated stem tips. This low growth point evolved in response to grazing animals and allows grasses to be grazed or mown regularly without severe damage to the plant.

Flowers of Poaceae are characteristically arranged in spikelets, each spikelet having one or more florets (the spikelets are further grouped into panicles or spikes). A spikelet consists of two (or sometimes fewer) bracts at the base, called glumes, followed by one or more florets. A floret consists of the flower surrounded by two bracts called the lemma (the external one) and the palea (the internal). The flowers are usually hermaphroditic (maize, monoecious, is an exception) and pollination is almost always anemophilous. The perianth is reduced to two scales, called lodicules, that expand and contract to spread the lemma and palea; these are generally interpreted to be modified sepals.

The fruit of Poaceae is a caryopsis in which the seed coat is fused to the fruit wall and thus, not separable from it (as in a maize kernel).

There are three general classifications of growth habit present in grasses; bunch-type (also called caespitose), stoloniferous and rhizomatous.

The success of the grasses lies in part in their morphology and growth processes, and in part in their physiological diversity. Most of the grasses divide into two physiological groups, using the C3 and C4 photosynthetic pathways for carbon fixation. The C4 grasses have a photosynthetic pathway linked to specialized Kranz leaf anatomy that particularly adapts them to hot climates and an atmosphere low in carbon dioxide.

C3 grasses are referred to as "cool season grasses" while C4 plants are considered "warm season grasses". Grasses may be either annual or perennial. Examples of annual cool season are wheat, rye, annual bluegrass (annual meadowgrass, Poa annua and oat). Examples of perennial cool season are orchard grass (cocksfoot, Dactylis glomerata), fescue (Festuca spp), Kentucky Bluegrass and perennial ryegrass (Lolium perenne). Examples of annual warm season are corn, sudangrass and pearl millet. Examples of Perennial Warm Season are big bluestem, indiangrass, bermuda grass and switch grass.

One classification of the grass family recognizes twelve subfamilies: These are 1) anomochlooideae, a small lineage of broad-leaved grasses that includes two genera (Anomochloa, Streptochaeta); 2) Pharoideae, a small lineage of grasses that includes three genera, including Pharus and Leptaspis; 3) Puelioideae a small lineage that includes the African genus Puelia; 4) Pooideae which includes wheat, barley, oats, brome-grass (Bronnus) and reed-grasses (Calamagrostis); 5) Bambusoideae which includes bamboo; 6) Ehrhartoideae, which includes rice, and wild rice; 7) Arundinoideae, which includes the giant reed and common reed; 8) Centothecoideae, a small subfamily of 11 genera that is sometimes included in Panicoideae; 9) Chloridoideae including the lovegrasses (Eragrostis, ca. 350 species, including teff), dropseeds (Sporobolus, some 160 species), finger millet (Eleusine coracana (L.) Gaertn.), and the muhly grasses (Muhlenbergia, ca. 175 species); 10) Panicoideae including panic grass, maize, sorghum, sugar cane, most millets, fonio and bluestem grasses; 11) Micrairoideae and 12) Danthoniodieae including pampas grass; with Poa which is a genus of about 500 species of grasses, native to the temperate regions of both hemispheres.

Agricultural grasses grown for their edible seeds are called cereals. Three common cereals are rice, wheat and maize (corn). Of all crops, 70% are grasses.

Sugarcane is the major source of sugar production. Grasses are used for construction. Scaffolding made from bamboo is able to withstand typhoon force winds that would break steel scaffolding. Larger bamboos and Arundo donax have stout culms that can be used in a manner similar to timber, and grass roots stabilize the sod of sod houses. Arundo is used to make reeds for woodwind instruments, and bamboo is used for innumerable implements.

Another naturally occurring ligno-cellulosic biomass feedstock may be woody plants or woods. A woody plant is a plant that uses wood as its structural tissue. These are typically perennial plants whose stems and larger roots are reinforced with wood produced adjacent to the vascular tissues. The main stem, larger branches, and roots of these plants are usually covered by a layer of thickened bark. Woody plants are usually either trees, shrubs, or lianas. Wood is a structural cellular adaptation that allows woody plants to grow from above ground stems year after year, thus making some woody plants the largest and tallest plants.

These plants need a vascular system to move water and nutrients from the roots to the leaves (xylem) and to move sugars from the leaves to the rest of the plant (phloem). There are two kinds of xylem: primary that is formed during primary growth from procambium and secondary xylem that is formed during secondary growth from vascular cambium.

What is usually called "wood" is the secondary xylem of such plants.

The two main groups in which secondary xylem can be found are:
1) conifers (Coniferae): there are some six hundred species of conifers. All species have secondary xylem, which is relatively uniform in structure throughout this group. Many conifers become tall trees: the secondary xylem of such trees is marketed as softwood.
2) angiosperms (Angiospermae): there are some quarter of a million to four hundred thousand species of angiosperms. Within this group secondary xylem has not been found in the monocots (e.g. Poaceae). Many non-monocot angiosperms become trees, and the secondary xylem of these is marketed as hardwood.

The term softwood is used to describe wood from trees that belong to gymnosperms. The gymnosperms are plants with naked seeds not enclosed in an ovary. These seed "fruits" are considered more primitive than hardwoods. Softwood trees are usually evergreen, bear cones, and have needles or scale like leaves. They include conifer species e.g. pine, spruces, firs, and cedars. Wood hardness varies among the conifer species.

The term hardwood is used to describe wood from trees that belong to the angiosperm family. Angiosperms are plants with ovules enclosed for protection in an ovary. When fertilized, these ovules develop into seeds. The hardwood trees are usually broad-leaved; in temperate and boreal latitudes they are mostly deciduous, but in tropics and subtropics mostly evergreen. These leaves can be either simple (single blades) or they can be compound with leaflets attached to a leaf stem. Although variable in shape all hardwood leaves have a distinct network of fine veins. The hardwood plants include e.g. Aspen, Birch, Cherry, Maple, Oak and Teak.

Therefore a preferred naturally occurring ligno-cellulosic biomass may be selected from the group consisting of the grasses and woods. Another preferred naturally occurring ligno-cellulosic biomass can be selected from the group consisting of the plants belonging to the conifers, angiosperms, Poaceae and families. Another preferred naturally occurring ligno-cellulosic biomass may be that biomass having at least 10% by weight of it dry matter as cellulose, or more preferably at least 5% by weight of its dry matter as cellulose.

### Pre-treatment

According to the invention ligno-cellulosic biomass is pre-treated. The term "pre-treated" may be replaced with the term "treated". However, preferred techniques contemplated are those well known for "pre-treatment" of ligno-cellulosic biomass as will be describe further below.

As mentioned above treatment or pre-treatment may be carried out using conventional methods known in the art, which promotes the separation and/or release of cellulose and increased accessibility of the cellulose from ligno-cellulosic biomass. Pre-treatment techniques are well known in the art and include physical, chemical, and biological pre-treatment, or any combination thereof. In preferred embodiments the pre-treatment of ligno-cellulosic biomass is carried out as a batch or continuous process.

Physical pre-treatment techniques include various types of milling/comminution (reduction of particle size), irradiation, steaming/steam explosion, and hydrothermolysis, in the preferred embodiment, soaking, removal of the solids from the liquid, steam exploding the solids to create the pre-treated ligno-cellulosic biomass.

Comminution includes dry, wet and vibratory ball milling. Preferably, physical pre-treatment involves use of high pressure and/or high temperature (steam explosion). In context of the invention high pressure includes pressure in the range from 3 to 6 MPa preferably 3.1 MPa. In context of the invention high temperature include temperatures in the range from about 100 to 300°C, preferably from about 160 to 235 °C. In a specific embodiment impregnation is carried out at a pressure of about 3.1 MPa and at a temperature of about 235 °C. In a preferred embodiment the physical pre-treatment is done according to the process described in WO 2010/113129.

Although not needed or preferred, chemical pre-treatment techniques include acid, dilute acid, base, organic solvent, lime, ammonia, sulfur dioxide, carbon dioxide, pH-controlled hydrothermolysis, wet oxidation and solvent treatment.

If the chemical treatment process is an acid treatment process, it is more preferably, a continuous dilute or mild acid treatment, such as treatment with sulfuric acid, or another organic acid, such as acetic acid, citric acid, tartaric acid, succinic acid, or any mixture thereof. Other acids may also be used. Mild acid treatment means at least in the context of the invention that the treatment pH lies in the range from 1 to 5, preferably 1 to 3.

In a specific embodiment the acid concentration is in the range from 0.1 to 2.0 % wt acid, preferably sulfuric acid. The acid is mixed or contacted with the ligno-cellulosic biomass and the mixture is held at a temperature in the range of around 160-220 °C for a period ranging from minutes to seconds. Specifically the pre-treatment conditions may be the following: 165-183 °C, 3-12 minutes, 0.5-1.4% (w/w) acid concentration, 15-25, preferably around 20% (w/w) total solids concentration. Other contemplated methods are described in U.S. Pat. Nos. 4,880,473, 5,366,558, 5,188,673, 5,705,369 and 6,228,177.

Wet oxidation techniques involve the use of oxidizing agents, such as sulfite based oxidizing agents and the like. Examples of solvent treatments include treatment with DMSO (Dimethyl Sulfoxide) and the like. Chemical treatment processes are generally carried out for about 5 to about 10 minutes, but may be carried out for shorter or longer periods of time.

Biological pre-treatment techniques include applying lignin-solubilizing micro-organisms (see, for example, Hsu, T.-A., 1996, Pre-treatment of biomass, in Handbook on Bioethanol: Production and Utilization, Wyman, C. E., ed., Taylor & Francis, Washington, D.C., 179-212; Ghosh, P., and Singh, A., 1993, Physicochemical and biological treatments for enzymatic/microbial conversion of ligno-cellulosic biomass, Adv. Appl. Microbiol. 39: 295-333; McMillan, J. D., 1994, Pretreating ligno-cellulosic biomass: a review, in Enzymatic Conversion of Biomass for Fuels Production, Himmel, M. E., Baker, J. O., and Overend, R. P., eds., ACS Symposium Series 566, American Chemical Society, Washington, D.C., chapter 15; Gong, C. S., Cao, N. J., Du, J., and Tsao, G. T., 1999, Ethanol production from renewable resources, in Advances in Biochemical Engineering/Biotechnology, Scheper, T., ed., Springer-Verlag Berlin Heidelberg, Germany, 65: 207-241; Olsson, L., and Hahn-Hagerdal, B., 1996, Fermentation of ligno-cellulosic hydrolysates for ethanol production, Enz. Microb. Tech. 18: 312-331; and Vallander, L., and Eriksson, K.-E. L., 1990, Production of ethanol from ligno-cellulosic materials: State of the art, Adv. Biochem. Eng./Biotechnol. 42: 63-95).

In an embodiment both chemical and physical pre-treatment is carried out including, for example, both mild acid treatment and high temperature and pressure treatment. The chemical and physical treatment may be carried out sequentially or simultaneously.

In a preferred embodiment the pre-treatment is carried out as a soaking step with water at greater than 1 °C, removing the ligno-cellulosic biomass from the water, followed by a steam explosion step.

In a preferred embodiment the pre-treated ligno-cellulosic biomass is comprised of complex sugars, also known as glucans and xylans (cellulose and hemicellulose) and lignin.

### Enzyme or enzyme mixture

An enzyme or enzyme mixture means a cellulolytic enzyme or mixture of enzymes capable of degrading ligno-cellulosic biomass. An enzyme or enzyme mixture produced according to the described process may be of any origin including of bacterial or fungal origin. Chemically modified or protein engineered variants are included. Suitable enzyme or enzyme mixtures include enzyme or enzyme mixtures from the general Cellulomonas, Bacillus, Pseudomonas, Humicola, Fusarium, Thielavia, Acremonium, Chrysosporium, Penicillium, Themobifida and Trichoderma, e.g., fungal enzyme or enzyme mixtures produced by Humicola insolens, Themobifida fusca, Cellulomonas fimi, Myceliophthora thermophila, Thielavia terrestris, Fusarium oxysporum, Chrysosporium lucknowense, Penicillium decumbens, and Trichoderma reesei.

In an embodiment the enzyme or enzyme mixture produced is an enzyme or enzyme mixture complex homologous to the host cell. In an embodiment the enzyme or enzyme mixture produced is an enzyme or enzyme mixture complex homologous to a host cell of the genus Penicillium, preferably a strain of Penicillium decumbens.

It is to be understood that the enzyme or enzyme mixture produced may also be a mono-component enzyme or enzyme mixture, e.g., comprise an endoglucanase, exo-cellobiohydrolase, glucohydrolase, or beta-glucosidase produced recombinantly in a suitable host cell. The enzyme or enzymes mixture may also include other enzymes or enzyme classes produced in recombinant cells exploiting at least a part of the coding region of the enzymes previously listed including the regulating and/or the promotor regions as known in the art. Suitable host cells are described further below.

The enzyme or enzyme mixture produced may also be an enzyme or enzyme mixture preparation where one or more homologous enzyme or enzyme mixture components are deleted or inactivated from the host cell natively producing the enzyme or enzyme mixture.

### Host Cell Capable of Producing an Enzyme or Enzyme Mixture

The host cell may be of any origin. As mentioned above the enzyme or enzyme mixture may be homologous or heterologous to the host cell capable of producing the enzyme or enzyme mixture.

The term "recombinant host cell", as used herein, means a host cell which harbours gene(s) encoding enzyme or enzyme mixture and is capable of expressing said gene(s) to produce enzyme or enzyme mixture, wherein the enzyme or enzyme mixture coding gene(s) have been transformed, transfected, transduced, or the like, into the host cell. The transformation, transfection, transduction or the like technique used may be well known in the art. In a preferred embodiment the gene is integrated into the genome of the recombinant host cell in one or more copies.

When the enzyme or enzyme mixture is heterologous the recombinant host cell capable of producing the enzyme or enzyme mixture is preferably of fungal or bacterial origin. The choice of recombinant host cell will to a large extent depend upon the gene(s) coding for the enzyme or enzyme mixture and the origin of the enzyme or enzyme mixture.

The term "wild-type host cell", as used herein, refers to a host cell that natively harbors gene(s) coding for enzyme or enzyme mixture and is capable of expressing said gene(s). When the enzyme or enzyme mixture is a homologous preparation or enzyme or enzyme mixture complex the wild-type host cell or mutant thereof capable of producing the enzyme or enzyme mixture is preferably of fungal or bacterial origin.

A "mutant thereof" may be a wild-type host cell in which one or more genes have been deleted or inactivated, e.g., in order to enrich the enzyme or enzyme mixture preparation in a certain component. A mutant host cell may also be a wild-type host cell transformed with one or more additional genes coding for additional enzymes or proteins in order to introduce one or more additional enzyme activities or other activities into the enzyme or enzyme mixture complex or preparation natively produced by the wild-type host cell. The additional enzyme(s) may have the same activity (e.g., enzyme or enzyme mixture activity) or merely be another enzyme molecule, e.g., with different properties. The mutant wild-type host cell may also have additional homologous enzyme coding genes transformed, transfected, transduced, or the like, preferably integrated into the genome, in order to increase expression of that gene to produce more enzyme.

In a preferred embodiment the recombinant or wild-type host cell is of filamentous fungus origin. Examples of host cells include the ones selected from the group comprising Acremonium, Aspergillus, Aureobasidium, Bjerkandera, Ceriporiopsis, Chrysosporium, Coprinus, Coriolus, Cryptococcus, Filobasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes, or Trichoderma cell.

In a more preferred embodiment the filamentous fungal host cell is selected from the group comprising a strain of Aspergillus awamori, Aspergillus fumigatus, Aspergillus foetidus, Aspergillus japonicus, Aspergillus nidulans, Aspergillus niger or Aspergillus oryzae. In an even more preferred embodiment, the strain is Penicillium decumbens.

In another preferred embodiment the filamentous fungal host cell is a strain of Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, or Fusarium venenatum cell. In another preferred embodiment, the filamentous fungal host cell is selected from the group comprising a strain of Bjerkandera adusta, Ceriporiopsis aneirina, Ceriporiopsis aneirina, Ceriporiopsis caregiea, Ceriporiopsis gilvescens, Ceriporiopsis pannocinta, Ceriporiopsis rivulosa, Ceriporiopsis subrufa, or Ceriporiopsis subvermispora, Chrysosporium lucknowense, Coprinus cinereus, Coriolus hirsutus, Humicola insolens, Humicola lanuginosa, Mucor miehei, Myceliophthora thermophila, Neurospora crassa, Penicillium purpurogenum, Penicillium decumbens, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei, or Trichoderma viride.

In another preferred embodiment the recombinant or wild-type host cell is of bacterial origin. Examples of host cells include the ones selected from the group comprising gram positive bacteria such as a strain of Bacillus, e.g., Bacillus alkalophilus, Bacillus amyloliquefaciens, Bacillus brevis, Bacillus circulans, Bacillus coagulans, Bacillus lautus, Bacillus lentus, Bacillus licheniformis, Bacillus megaterium, Bacillus stearothermophilus, Bacillus subtilis, or Bacillus thuringiensis; or a Streptomyces strain, e.g., Streptomyces lividans or Streptomyces murinus; or from a gram negative bacterium, e.g., E. coli or Pseudomonas sp.

### Hydrolysis

The biomass will contain some compounds which are hydrolysable into a watersoluble species obtainable from the hydrolysis of the biomass. In the case of watersoluble hydrolyzed species of cellulose, cellulose can be hydrolyzed into glucose, cellobiose, and higher glucose polymers and includes dimers and oligomers. Thus some of the water-soluble hydrolyzed species of cellulose are glucose, cellobiose, and higher glucose polymers and includes their respective dimers and oligomers. Cellulose is hydrolysed into glucose by the carbohydrolytic cellulases. Thus the carbohydrolytic cellulases are examples of catalysts for the hydrolysis of cellulose.

The prevalent understanding of the cellulolytic system divides the cellulases into three classes; exo-1,4-[beta]-D-glucanases or cellobiohydrolases (CBH) (EC 3.2.1.91), which cleave off cellobiose units from the ends of cellulose chains; endo-1,4-[beta]-D-glucanases (EG) (EC 3.2.1.4), which hydrolyse internal [beta]-1,4- glucosidic bonds randomly in the cellulose chain; 1,4-[beta]-D-glucosidase (EC 3.2.1.21), which hydrolyses cellobiose to glucose and also cleaves off glucose units from cellooligosaccharides. Therefore, if the biomass contains cellulose, then glucose is a water-soluble hydrolyzed species obtainable from the hydrolysis of the biomass and the afore mentioned cellulases are specific examples, as well as those mentioned in the experimental section, of catalysts for the hydrolysis of cellulose.

By similar analysis, the hydrolysis products of hemicellulose are water-soluble species obtainable from the hydrolysis of the biomass, assuming of course, that the biomass contains hemicellulose. Hemicellulose includes xylan, glucuronoxylan, arabinoxylan, glucomannan, and xyloglucan. The different sugars in hemicellulose are liberated by the hemicellulases. The hemicellulytic system is more complex than the cellulolytic system due to the heterologous nature of hemicellulose. The systems may involve among others, endo-1,4-[beta]-D-xylanases (EC 3.2.1.8), which hydrolyse internal bonds in the xylan chain; 1 ,4-[beta]-D-xylosidases (EC 3.2.1.37), which attack xylooligosaccharides from the non- reducing end and liberate xylose; endo-1,4-[beta]-D-mannanases (EC 3.2.1.78), which cleave internal bonds; 1,4-[beta]-D-mannosidases (EC 3.2.1.25), which cleave mannooligosaccharides to mannose. The side groups are removed by a number of enzymes; such as [alpha]-D- galactosidases (EC 3.2.1.22), [alpha]-L-arabinofuranosidases (EC 3.2.1.55), [alpha]-D-glucuronidases (EC 3.2.1.139), cinnamoyl esterases (EC 3.1.1.-), acetyl xylan esterases (EC 3.1.1.6) and feruloyl esterases (EC 3.1.1.73). Therefore, if the biomass contains hemicellulose, then xylose and mannose are examples of a watersoluble hydrolyzed species obtainable from the hydrolysis of the hemicellulose containing biomass and the afore mentioned hemicellulases are specific examples, as well as those mentioned in the experimental section, of catalysts for the hydrolysis of hemicellulose.

Included in the process is a hydrolysis catalyst. The hydrolysis catalyst composition consists of the catalyst, the carrier, and other additives/ingredients used to introduce the catalyst to the process. As discussed above, the catalyst may comprise at least one enzyme or microorganism which converts at least one of the compounds in the biomass to a compound or compounds of lower molecular weight, down to, and including, the basic sugar or carbohydrate used to make the compound in the biomass. The enzymes capable of doing this for the various polysaccharides such as cellulose, hemicellulose, and starch are well known in the art and would include those not invented yet.

The hydrolysis catalyst composition may also comprise an inorganic acid preferably selected from the group consisting of sulfuric acid, hydrochloric acid, phosphoric acid, and the like, or mixtures thereof. The inorganic acid is believed useful for processing at temperatures greater than 100 °C. The process may also be run specifically without the addition of an inorganic acid.

It is typical to add the hydrolysis catalyst to the process with a carrier, such as water or an organic based biomass. For mass balance purposes, the term catalyst composition therefore includes the catalyst(s) plus the carrier(s) used to add the catalyst(s) to the process. If a pH buffer is added with the catalyst, then it is part of the catalyst composition as well. Often the ligno-cellulosic biomass will contain starch. The more important enzymes for use in starch hydrolysis are alpha-amylases (1,4-(alpha)-D-glucan glucanohydrolases, (EC 3.2.1.1)). These are endo-acting hydrolases which cleave 1,4-[alpha]-D-glucosidic bonds and can bypass but cannot hydrolyse 1,6-[alpha)-D-glucosidic branchpoints. However, also exo-acting glycoamylases such as beta-amylase (EC 3.2.1.2) and pullulanase (EC 3.2.1.41) can be used for starch hydrolysis. The result of starch hydrolysis is primarily glucose, maltose, maltotriose, [alpha]-dextrin and varying amounts of oligosaccharides. When the starch-based hydrolysate is used for fermentation it can be advantageous to add proteolytic enzymes. Such enzymes may prevent flocculation of the microorganism and may generate amino acids available to the microorganism. Therefore, if the biomass contains starch, then glucose, maltose, maltotriose, [alpba]-dextrin and oligosaccharides are examples of a water-soluble hydrolyzed species obtainable from the hydrolysis of the starch containing biomass and the afore mentioned alpha-amylases are specific examples, as well as those mentioned in the experimental section, of catalysts for the hydrolysis of starch.

### Use

The process may also have additional steps wherein the enzymes harvested from the process are further used to hydrolyze a first ligno-cellulosic biomass. Preferably first ligno-cellulosic biomass and second ligno-cellulosic biomass should be derived from the same grass genus and more preferably derived from the same grass species. It is also preferable that the first ligno-cellulosic biomass upon which the enzymatic hydrolysis to is to be conducted be pre-treated prior to enzymatic hydrolysis.

### EXPERIMENTAL PROCEDURE

### PREPARATION OF SOLID COMPOSITIONS

Different solid compositions were prepared from Arundo donax and wheat straw. The biomasses were subjected to pre-treatment, enzymatic hydrolysis, fermentation and press filtration for preparing different solid compositions used in cultivation experiments.

### Pre-treatment

Ligno-cellulosic biomass was introduced into a continuous reactor and subjected to a soaking treatment. The soaked mixture was separated in a soaked liquid and a fraction containing the solid soaked raw material by means of a press. The fraction containing the solid soaked raw material was subjected to steam explosion. Pretreatment parameters for the two biomasses are reported in Table 1.

**TABLE 1: Process parameters used in the pre-treatment**

| | **Soaking** | | **Steam explosion** | |
|---|---|---|---|---|
| | **Temperature (°C)** | **Time (minutes)** | **Temperature (°C)** | **Time (minutes)** |
| Arundo donax | 155 | 155 | 195 | 4 |
| Wheat straw | 155 | 65 | 190 | 4 |

Steam exploded products were separated into a steam explosion liquid and a steam exploded solid.

Steam exploded solid is the Arundo donax pretreated biomass (AR) and the wheat straw pretreated biomass (WS) used in the cultivation experiments.

### Enzymatic hydrolysis

Pretreated biomass was mixed with water to obtain a mixture having 7.5% dry matter content and the mixture was inserted into an enzymatic reactor. An enzyme cocktail was added, corresponding to the specified concentration of protein per gram of glucans contained in the pretreated biomass. Enzymatic hydrolysis was carried out for 72 hours under agitation.

Two different hydrolyzates were prepared starting from Wheat Straw pretreated biomass; each hydrolyzate was separated by centrifugation (20 minutes, 8000 rpm, 4°C) in a liquid fraction, containing the water-soluble sugars, and the solid composition. Solid compositions, indicated as HW1 and HW2, were used in the the cultivation experiments. Hydrolysis parameters used in the preparation of HW1 and HW2 are reported in Table 2 and were chosen to obtain two different complex sugar depletion levels; in particular HW2 was prepared with a higher enzyme concentration, thereby corresponding to a more depleted composition.

**TABLE 2. Parameters used in enzymatic hydrolysis for producing HW1 and HW2 solid compositions**

| | Enzyme | Concentration (mg protein /g glucan) | Temperature (°C) | pH | Time (hours) |
|---|---|---|---|---|---|
| HW1 | T. reesei enzyme solution | 15 | 60 | 5 | 72 |
| HW2 | T. reesei enzyme solution | 75 | 50 | 5 | 72 |

### Fermentation and press filtration

Wheat straw hydrolyzate, prepared according to the conditions of composition HW2, was inserted into a bioreactor with 3 g/l urea and 0.5 g/l of a fermenting yeast. pH was set to 5 and temperature to 30 °C and fermentation carried out for 48 hours. Enzymatic cocktail was not removed from the hydrolyzate, in such a way that the enzymatic hydrolysis continued during fermentation. The fermentation broth, comprising solids, ethanol and other liquid fractions, was pressed by a press filtering at a temperature of 80 °C at 15 bar, for separating ethanol and liquid components from the solid-rich fraction. The solid-rich fraction comprising lignin extracted from the press, indicated as HFW, was used in the cultivation experiments.

Arundo Donax hydrolyzate was prepared according to parameters corresponding to HW2 and fermented according to the procedure used for wheat straw. The solid-rich fraction extracted from the press, indicated as HFA, was used in the cultivation experiments.

### Composition

Composition of pre-treated biomass and solid compositions in terms of water soluble sugars, complex sugars, lignin and other compounds was determined according to standard analytical methods listed at the end of experimental section.

Table 3 reports the composition of pretreated biomass AR and WS, solid compositions derived from WS hydrolyzates HW1 and HW2 and solid compositions derived from fermentation of AR and WS hydrolyzates (HFW and HFA). The table contains also the compositions of LS1 and LS2, which were used in following experiments and are described in a following experimental section.

The compositions are expressed in terms of complex C6 sugars, complex C5 sugars, total water soluble sugars (C5 and C6) and other components (comprising lignin and water). Complex C6 sugars are based on glucose, complex C5 sugars are based mainly on xylose.

**TABLE 3. Compositions used in the reported experiments**

| | **WS** | **AR** | **HW1** | **HW2** | **HFW** | **HFA** | **LS1** | **LS2** |
|---|---|---|---|---|---|---|---|---|
| **Water soluble sugars** | 1.6% | 2.4% | 0.0% | 0.0% | 0.0% | 0.0% | 0.0% | 0.0% |
| **Complex C6 sugars** | 17.4% | 18.3% | 10.7% | 9.6% | 8.05% | 14.0% | 0.51% | 0.23% |
| **Complex C5 sugars** | 3.4% | 2.4% | 1.6% | 1.4% | 1.3% | 2.0% | 0.04% | 0.02% |
| **Other*** | 77.6% | 76.9% | 87.7% | 89.0% | 90.6% | 84.0% | 99.4% | 99.8% |
| **Complex sugar/ lignin ratio** | 1.831 | 1.815 | 1.574 | 0.886 | 0.723 | 0.786 | 0.19 | 0.18 |

| | | | | | | | | |
|---|---|---|---|---|---|---|---|---|
| *Including lignin and water | | | | | | | | |

For the scope of the present disclosure, the compositions are conveniently characterized by the complex sugars to lignin ratio. Complex sugars correspond to the sum of complex C6 sugars and complex C5 sugars. Considering the compositions obtained from WS, that is HW1, HW2 and HFW, it is noted that:
- complex sugars to lignin ratio in HW1 and HW2 is lower than complex sugars to lignin ratio in WS, reflecting the complex sugar-depletion occurred in the hydrolysis;
- complex sugars to lignin ratio in HW2 is lower than complex sugars to lignin ratio in HW1, reflecting the more effective hydrolysis in HW2 performed at higher enzyme concentration;
- complex sugars to lignin ratio in HFW is lower than complex sugars to lignin ratio in HW2, due to the fact that enzymes were not deactivated and removed and hydrolysis continued during fermentation

### CULTIVATION EXPERIMENTS

The cultivation of the host cell for the production of enzymes proceeded as in the following examples. Each host cell cultivation, which in the reported examples used Trichoderma reesei and Penicillium decumbens as the host cell started from a spore solution recovered from a PDA-plate seeded with fresh spores seven days before recovery.

Experiments are divided in two steps:
1) pre-cultivation which is not part of the claimed cultivation process and
2) host cell cultivation wherein the host cell is grown and the enzyme(s) produced.

### PRE-CULTIVATION:

### Seeding PDA plate:

1. 500 µl of a previously collected spore solution were dispensed into a PDA plate (3.9% Potato Dextrose Agar medium) prepared as known in the art.
2. 500 µl of sterile 0.9% NaCl solution for P. decumbens or 1% Triton X-100 solution for T. reesei were dispensed over the spores and the flask gently rotated until the surface was all covered by the liquid.
3. The flask was closed with a cotton plug covered with an aluminium foil and incubated at 30 °C for P. decumbens or 28 °C for T. reesei for 7 days.

### Spore Solution Recovery:

4. After 7 days, 10 ml of the proper sterile solution for P. decumbens and T. reesei (0.9% NaCl and 1% Triton X-100 respectively) were dispensed in the flask,.
5. The flask was gently rotated until the liquid became cloudy.
6. As much volume of the dispensed solution was drawn back as possible removing the spore suspension to a sterile tube.
7. The spore solution was stored at 4 °C.

### Pre-cultivation Setup Step

Before starting the host cell cultivation it was necessary to set up the pre-cultivation.

Pre-culture medium was prepared as reported in table 4, choosing the appropriate volume with respect to that of the host cell cultivation phase (one-tenth and one-twentieth for P. decumbens and T. reesei respectively)

**TABLE 4: Pre-culture medium composition**

| **P. decumbens** | | | | **T. reesei** | | | |
|---|---|---|---|---|---|---|---|
| **Pre-Cultivation Medium Composition** | **% w/v** | **g** | **G** | **Pre-Cultivation Medium Composition** | **% w/v** | **g** | **G** |
| Peptone | 1.00% | 1.00 | 10.00 | (NH₄)₂SO₄ | 0.14% | 0.14 | 1.4 |
| AR | 2.00% | 2.00 | 20.00 | KH₂PO₄ | 0.21% | 0.21 | 2.1 |
| KH₂PO₄ | 0.30% | 0.30 | 3.00 | CaCl₂ 2H₂O | 0.04% | 0.04 | 0.4 |
| MgSO₄ | 0.05% | 0.05 | 0.50 | MgSO₄ 7H₂O | 0.04% | 0.04 | 0.4 |
| (NH₄)₂SO₄ | 0.20% | 0.20 | 2.00 | Metals | 0.02% | 0.02 | 0.2 |
| CaCO₃ | 0.50% | 0.50 | 5.00 | Urea | 0.10% | 0.1 | 1 |
| Glucose | 1.00% | 1.00 | 10.00 | Glucose | 1.00% | 1 | 10 |
| Final volume | | 100 | 1000 | Final volume | | 100 | 1000 |

1. The glucose and spore solution were added after sterilization. Spore solution volume was chosen to obtain a final concentration of 5000 CFU/ml for P. decumbens and 50000 CFU/ml for T. reesei.
2. P. decumbens pre-culture was incubated at 30 °C, 170 rpm for 30 h while T. reesei pre-culture was incubated at 28 °C, 170 rpm for 64 h.

### HOST CELL CULTIVATION AND ENZYME PRODUCTION

The host cell cultivation environment is prepared as reported in the table 5.

**TABLE 5: Cultivation medium composition**

| **P. decumbens** | | **T. reesei** | |
|---|---|---|---|
| **Cultivation Medium Composition** | **% w/v** | **Cultivation Medium Composition** | **% w/v** |
| Biomass [on a dry basis] | 4.50% | Biomass [on a dry basis] | 4.0% |
| Urea | 0.50% | Urea | 0.18% |
| Tween | 0.10% | CaCl₂ | 0.26% |
| Pre-Culture Medium volume from previous step | 10.0% | KH₂PO₄ | 0.28% |
| | | MgSO₄ | 0.16% |
| | | (NH₄)₂SO₄ | 0.38% |
| | | Pre-Culture Medium volume from previous step | 5.0% |

The biomass is selected from the group of compositions prepared for demonstrating the invention (indicated as WS, AR, HW1, HW2, HFW, HFA).
1. After sterilization the pH was corrected to 5.3 for P. decumbens and 6.0 for T. reesei and pre-culture volumes were added. The pH is controlled in flasks using different type of buffer solutions (for example 0.1 M phosphate buffer).
2. Host cell cultivation and enzyme production were carried out at the proper temperature and agitation for each microorganism (30 °C, 170 rpm for P. decumbens and 28 °C, 170 rpm for T. reesei).

### EXPERIMENT 1

**Table 6: Enzyme activity of T. reesei in the presence of different cultivation media.**

| | **Composition used** | **Concentration in cultivation environment ((w/v, %)** | | **Total Cellulasic, FPU** | **β-Glucosidase** | **Xylanase** |
|---|---|---|---|---|---|---|
| | | **Lignin** | **Complex sugar** | | | |
| | | | | **Activity content after 169 hours, U/ml** | | |
| CE1 | WS | 1.65 | 3.02 | 0.96 | 0.30 | 231 |
| WE1 | HW1 | 1.67 | 2.63 | 1.92 | 0.45 | 113 |
| WE2 | HW2 | 1.72 | 1.52 | 1.74 | 0.46 | 138 |
| WE3 | HFW | 1.89 | 1.35 | 2.01 | 0.91 | 360 |

| | | | | **Relative performance to CE1, %** | | |
|---|---|---|---|---|---|---|
| CE1 | WS | -- | -- | -- | -- | -- |
| WE1 | HW1 | 101% | 87% | 199% | 149% | 49% |
| WE2 | HW2 | 104% | 50% | 181% | 155% | 60% |
| WE3 | HFW | 114% | 45% | 216% | 305% | 156% |

Table 6 compares activity upon different ligno-cellulosic biomasses used to feed the host cell, Trichoderma reesei. In the table, "composition used" indicates the biomass used in the cultivation medium as the source of carbon atoms. Medium content was calculated to provide approximately the same amount of lignin in each cultivation environment. As each used biomass is characterized by decreasing complex sugar to lignin ratio, the amount of complex sugar used in each cultivation media decreases from CE1 to WE3. This establishes the method for various types of host cells. CE1 activities were chosen as reference for expressing experimental results on a relative scale in the second section of the table. WE1, WE2 and WE3 experiments used a cultivation medium where the only carbon source was the composition obtained from hydrolysed (HW1 and HW2) and fermented (HFW) pretreated biomass. Cultivation time was the same in all the experiments (169 hours).

Experimental results clearly show that the pre-treated ligno-cellulosic biomass could be substituted with a solid composition obtained from pretreated ligno-cellulosic biomass by removing a fraction of complex sugars. Moreover, conversion yield - defined as total activity produced to complex sugar ratio - obtained from sugar-depleted ligno-cellulosic biomass is surprisingly higher than that obtained from pre-treated ligno-cellulosic biomass with an increase in the conversion yield as demonstrated by results reported in table 7.

**Table 7. Total activity obtained from different medium prepared using different composition with decreasing complex sugar content.**

| | **Complex sugar content in the cultivation medium relative to CE1** | **Conversion yield (tot activity /complex sugar)** | | |
|---|---|---|---|---|
| | | Total Cellulasic, FPU | β-Glucosidase | Xylanase |
| **CE1** | -- | 32 | 10 | 7649 |
| **WE1** | 87% | 73 | 17 | 4313 |
| **WE2** | 50% | 114 | 30 | 9059 |
| **WE3** | 45% | 149 | 67 | 26667 |

### EXPERIMENT 2

In a further experiment, different compositions having no or very low complex sugar content were used in the cultivation medium as carbon sources.

CE2 and CE3 were produced using two different commercial lignin samples. CE2 contained 8% of Lignin alkali low sulphonate content (Sigma, code 471003) and CE3 contained 8% of Lignin alkali (Sigma, code 370959). The commercial lignin used have a complex sugar content lower than 5%.

After the enzyme production process, the residues of the cultivation media used in two different WE3 experiments were centrifuged for 20 minutes at 8000 rpm and 4°C and two solid rich fractions, indicated respectively as LSI and LS2, were collected. CE1 from Experiment 1 is reported as control experiment.

Compositions of LSI and LS2 are contained in table 3. WE4 and WE5 cultivation media contained 5 times more material then CE1 with respect to lignin content and 2.5 - 2.8 times more material than LS1 and LS2 respectively. As evidenced in table 3, complex sugar content in LS1 and LS2 is less than 8% and the corresponding amount of complex sugar added to the medium is reported in the table.

Cultivation time was 169 hours.

**Table 8. Enzyme activity of T. reesei for CE1, CE2, CE3, WE4 and WE5 experiments.**

| | **Carbon source** | **Concentration in cultivation environment (%, dry basis)** | | **Total Cellulasic, FPU** | **β-Glucosidase** | **Xylanase** |
|---|---|---|---|---|---|---|
| | | **Lignin** | **Complex sugar** | | | |
| | | | | **Activity content after 169 hours, U/ml** | | |
| **CE1** | WS | 1.65 | 3.02 | 2.57 | 1.20 | 329.96 |
| **CE2** | Pure Lignin | 7.92 | 0 | 0.13 | 0.09 | 13.04 |
| **CE3** | Pure Lignin | 7.92 | 0 | 0.07 | 0.11 | 13.01 |
| **WE4** | LS1 | 3.04 | 0.59 | 0.02 | 0.00 | 10.94 |
| **WE5** | LS2 | 2.79 | 0.50 | 0.19 | 0.00 | 26.01 |

| | | | | **Relative performance to CE1, %** | | |
|---|---|---|---|---|---|---|
| **CE1** | WS | -- | -- | -- | -- | -- |
| **CE2** | Pure Lignin | 480% | 0% | 5% | 8% | 4% |
| **CE3** | Pure Lignin | 480% | 0% | 3% | 9% | 4% |
| **WE4** | LS1 | 184% | 20% | 1% | 0% | 3% |
| **WE5** | LS2 | 169% | 17% | 7% | 0% | 8% |

As it appears clear from data reported, in the case of WE4 and WE5 the complex sugar amount, corresponding to 20% of complex sugars in CE1 case, is not sufficient to promote enzyme production, while the presence of 45% of the complex sugars used in CE1 were reported to increase the production of enzymes (WE3 experiment).

### EXPERIMENT 3

Further experiments were carried on to produce enzymes using Penicillium decumbens as the host cell. The comparative example (CE4) was conducted using AR pre-treated biomass as the carbon source while WE6 was conducted adding a sufficient amount of HFA composition to provide approximately the same amount of lignin used in CE4. Cultivation time was the same for all the experiments (138 hours).

Table 9 reports the activities obtained after 138h of enzyme production. The experiments show that at least the same amount of total enzymes could be produced when Penicillium decumbens is cultivated either in the presence of pre-treated or sugar-depleted ligno-cellulosic biomass.

**Table 9: Enzyme activity produced using Penicillium decumbens as the host cell**

| | **Carbon source** | **Concentration in cultivation environment (%, dry basis)** | | **Total Cellulasic, FPU** | **β-Glucosidase** | **Xylanase** |
|---|---|---|---|---|---|---|
| | | **Lignin** | **Complex Sugars** | | | |
| | | | | **Activity content after 138 hours, U/ml** | | |
| **CE4** | AR | 4.90 | 2.43 | 0.54 | 2.38 | 16.69 |
| **WE6** | HFA | 4.98 | 1.69 | 0.68 | 2.20 | 13.40 |

| | | | | **Relative performance to CE4, %** | | |
|---|---|---|---|---|---|---|
| **CE4** | AR | 100% | 100% | 100% | 100% | 100% |
| **WE6** | HFA | 102% | 70% | 126% | 92% | 80% |

It is apparent from Table 9, that the enzymes derived from the host cell fed with the sugar-depleted solid composition obtained from hydrolysis and fermentation of ligno-cellulosic biomasses are very close to those derived from the comparative example obtained using pre-treated material.

### EXPERIMENT 4

The enzymes produced in WE3 on wheat straw solid composition HFW were further used to hydrolyze wheat straw pre-treated biomass WS.

Pretreated biomass was mixed with water to obtain a mixture having 7.5% dry matter content and the mixture was inserted into a 100 ml flask. Enzyme mixtures grown in WE3 were loaded at 60 and 90 mg of protein/g glucans of the pre-treated biomass in WE7 and WE8 experiments respectively, and hydrolysis were carried out at pH 5.0 and 50°C for 72h. CE5 experiment was performed using a commercial enzyme solution following producer specifications.

Glucose and xylose concentrations were measured at 6, 24, 48 and 72 hours and are reported in Table 10. Glucose and xylose released were reported to the initial amount of glucans and xylans in the WS pre-treated biomass to quantify glucans hydrolysis yield and xylans hydrolysis yield. Figure 1 reports the graphs of glucans hydrolysis yield (Figure 1a) and xylans hydrolysis yield (Figure 1b). These experiment highlights that the enzymes produced according to the present disclosure can be used for effectively hydrolysing pre-treated ligno-cellulosic biomasses.

**Table 10. Hydrolysis results of CE5, WE7 and WE8 experiments.**

| | Glucans Hydrolysis performance | | | | | Xylans Hydrolysis performance | | | |
|---|---|---|---|---|---|---|---|---|---|
| Time, h | 6 | 24 | 48 | 72 | | 6 | 24 | 48 | 72 |

| | Glucose concentration, g/dm³ | | | | | Xylose concentration, g/dm³ | | | |
|---|---|---|---|---|---|---|---|---|---|
| CE5 | 7.0 | 18.6 | 22.0 | 23.2 | | 7.9 | 9.6 | 10.4 | 10.6 |
| WE7 | 8.5 | 18.0 | 20.2 | 23.5 | | 3.8 | 7.2 | 8.0 | 9.07 |
| WE8 | 10.7 | 20.6 | 23.3 | 24.5 | | 4.7 | 8.2 | 9.2 | 9.6 |

| | Glucans hydrolysis yield, % | | | | | Xylans hydrolysis yield, % | | | |
|---|---|---|---|---|---|---|---|---|---|
| CE5 | 24% | 64% | 75% | 80% | | 75% | 91% | 98% | 100% |
| WE7 | 29% | 62% | 69% | 81% | | 36% | 68% | 76% | 86% |
| WE8 | 37% | 71% | 80% | 84% | | 45% | 77% | 86% | 90% |

### ANALYTICAL METHODS

### Activity determination

The FPU (filter paper activity), Beta-glucosidase and Xylanase activity were determined using industry known methods of determining enzymatic activity. The difference being that filter paper was the substrate for FPU and salicin and the xylan mixture described below were used as the Beta-glucosidase and Xylanase activity assays.

| **Substrate** | **Activity type** | **Preparation** |
|---|---|---|
| Salicin | glucosidase | 0.1 g in 10 ml of 50 mM buffer solution |
| Xylan | Xylanase | 2 g Beechwood xylan |
| | | 70 ml Ultrapure water |
| | | Heat to boiling with stirring. Cool to room temperature and add 5 ml of 1 N buffer stock solution |

### Composition determination

Compositions were performed according to the following NREL standards

### NREL Analytical Method

### Determination of Structural Carbohydrates and Lignin in Biomass

Laboratory Analytical Procedure (LAP) Issue Date: 4/25/2008
Technical Report NREL/TP-510-42618 Revised April 2008

### Determination of Extractives in Biomass

Laboratory Analytical Procedure (LAP) Issue Date: 7/17/2005
Technical Report NREL/TP-510-42619 January 2008

### Preparation of Samples for Compositional Analysis

Laboratory Analytical Procedure (LAP) Issue Date: 9/28/2005
Technical Report NREL/TP-510-42620 January 2008

### Determination of Total Solids in Biomass and Total Dissolved Solids in Liquid Process Samples

Laboratory Analytical Procedure (LAP) Issue Date: 3/31/2008
Technical Report NREL/TP-510-42621 Revised March 2008

### Determination of Ash in Biomass

Laboratory Analytical Procedure (LAP) Issue Date: 7/17/2005
Technical Report NREL/TP-510-42622 January 2008

### Determination of Sugars, Byproducts, and Degradation Products in Liquid Fraction Process Samples

Laboratory Analytical Procedure (LAP) Issue Date: 12/08/2006
Technical Report NREL/TP-510-42623 January 2008

### Determination of Insoluble Solids in Pretreated Biomass Material

Laboratory Analytical Procedure (LAP) Issue Date: 03/21/2008
NREL/TP-510-42627 March 2008

## Claims

1. A process to produce at least a first enzyme capable of hydrolysing a first pre-treated ligno-cellulosic biomass, comprising the step of cultivating the host cell in a sugar-depleted cultivation environment comprising the host cell, water and a solid composition, obtained from a second pre-treated ligno-cellulosic biomass comprising a complex sugar selected from the group of polymeric water-insoluble glucans and xylans, and a lignin; wherein the ratio of the total amount of the complex sugars to the total amount of the lignin in the solid composition is less than the ratio of the total amount of the complex sugars to the total amount of the lignin in the second pre-treated ligno-cellulosic biomass;
and wherein said solid composition is obtained by removing a fraction, but not all, of the complex sugars contained in the second pre-treated ligno-cellulosic biomass.

2. The process of claim 1, wherein the cultivation is done under simple sugar-depleted conditions of having an amount of added simple sugar or simple sugars in the range of between 0 to 10% by weight of the sugar-depleted cultivation environment on a dry basis for a portion of the cultivation time which is at least 50% of the cultivation time; wherein said simple sugars are selected from monomeric sugars.

3. The process of claims 1 or 2, wherein the solid composition is obtained from the second pre-treated ligno-cellulosic biomass by a process comprising the steps of:
a) hydrolysing at least a fraction of the second pre-treated ligno-cellulosic biomass in the presence of a hydrolysis catalyst, to produce a hydrolysed composition comprising a solid composition and a solution of water and soluble sugars and;
b) removing at least 50% of the solution of water and soluble sugars from the hydrolysed composition.

4. The process of claim 3, wherein the removal of the solution of water and soluble sugars is done by at least one method selected from the group consisting of decantation, sedimentation, centrifugation, filtration, press filtration and washing.

5. The process of claims 1 or 2, wherein the solid composition is obtained from the second pre-treated ligno-cellulosic biomass by a process comprising the steps of:
a) hydrolysing at least a fraction of the second pre-treated ligno-cellulosic biomass in the presence of a hydrolysis catalyst, to produce a hydrolysed composition comprising said complex sugars and lignin and the solution of water and soluble sugars;
b) converting at least a fraction of the soluble sugars in the hydrolysed composition to produce a converted composition comprising at least one sugar-derived product, and the solid composition;
c) removing a fraction of the at least one sugar-derived product.

6. The process of claim 5, wherein the removal of the fraction of the at least one sugar-derived product is done by at least one method selected from the group consisting of decantation, sedimentation, centrifugation, filtration, press filtration, washing, evaporation and distillation.

7. The process of any of claims 3 to 6, wherein the hydrolysis catalyst comprises a second enzyme or enzymes mixture.

8. The process of any of claims 3 to 6, wherein the hydrolysis catalyst is selected from the group consisting of hydrogen ions, organic acids and inorganic acids.

9. The process of any of claims 5 to 8, wherein the conversion of the hydrolysed composition comprises a fermentation.

10. The process of claim 9, wherein the at least one sugar-derived product comprises ethanol.

11. The process of any of claims 2 to 10, wherein the portion of the cultivation time under simple sugar-depleted conditions is at least 75% of the cultivation time.

12. The process of any of claims 2 to 10, wherein the portion of the cultivation time under sugar-depleted conditions is the same as the cultivation time.

13. The process of any of claims 2 to 12, wherein the amount of added simple sugar or sugars is in the range of between 0 to 5% by weight of the sugar-depleted cultivation environment on a dry basis.

14. The process of any of claims 2 to 12, wherein there is no added simple sugar in the sugar-depleted cultivation environment.

15. The process of any of claims 1 to 14, wherein at least the first enzyme is harvested by separating the sugar-depleted cultivation environment into at least a harvested composition, comprising at least a fraction of the amount of the first enzyme, and an exhausted composition, comprising at least a fraction of an amount of solid residue of the solid composition.

16. The process of claim 15, wherein the total amount of said complex sugars in the exhausted composition is present in a range of 0 to 30% by weight on a dry basis.

17. The process of any of claims 1 to 16, wherein the first enzyme is further used to hydrolyse the first ligno-cellulosic biomass and the first ligno-cellulosic biomass and the second pre-treated ligno-cellulosic biomass both comprise ligno-cellulosic biomass derived from the group consisting of the same grass genus and the same grass species.

## Patentansprüche

1. Verfahren zur Herstellung mindestens eines ersten Enzyms, das in der Lage ist, eine erste vorbehandelte lignozellulosische Biomasse zu hydrolysieren, umfassend den Schritt der Kultivierung der Wirtszelle in einer zuckerabgereicherten Kultivierungsumgebung, umfassend die Wirtszelle, Wasser und eine feste Zusammensetzung, erhalten aus einer zweiten vorbehandelten lignozellulosischen Biomasse, umfassend einen Komplex-Zucker, ausgewählt aus der Gruppe der polymeren wasserunlöslichen Glucane und Xylane, und einem Lignin; wobei das Verhältnis der Gesamtmenge der Komplex-Zucker zu der Gesamtmenge des Lignins in der festen Zusammensetzung geringer ist als das Verhältnis der Gesamtmenge der Komplex-Zucker zu der Gesamtmenge des Lignins in der zweiten vorbehandelten lignozellulosischen Biomasse;
und wobei die feste Zusammensetzung erhalten wird durch Entfernen einer Fraktion, aber nicht aller, der Komplex-Zucker enthalten in der zweiten vorbehandelten lignozellulosischen Biomasse.

2. Verfahren nach Anspruch 1, wobei die Kultivierung unter einfachen zuckerabgereicherten Bedingungen durchgeführt wird mit einer Menge an zugesetztem einfachen Zucker oder einfachen Zuckern in einem Bereich zwischen 0 bis 10 Gew.-% der zuckerabgereicherten Kultivierungsumgebung, auf Trockenbasis, für einen Teil der Kultivierungszeit, die mindestens 50% der Kultivierungszeit ist; wobei die einfachen Zuckern ausgewählt sind aus monomeren Zuckern.

3. Verfahren nach Anspruch 1 oder 2, wobei die feste Zusammensetzung erhalten wird aus der zweiten vorbehandelten lignozellulosischen Biomasse durch ein Verfahren, umfassend die Schritte von:
a) Hydrolysieren mindestens einer Fraktion der zweiten vorbehandelten lignozellulosischen Biomasse in der Gegenwart eines Hydrolysekatalysators, um eine hydrolysierte Zusammensetzung zu erhalten, die eine feste Zusammensetzung und eine Lösung aus Wasser und löslichen Zuckern umfasst und;
b) Entfernen von mindestens 50% der Lösung von Wasser und löslichen Zuckern von der hydrolysierten Zusammensetzung.

4. Verfahren nach Anspruch 3, wobei die Entfernung der Lösung aus Wasser und löslichen Zuckern durchgeführt wird durch mindestens ein Verfahren ausgewählt aus der Gruppe, bestehend aus Dekantieren, Sedimentieren, Zentrifugieren, Filtrieren, Druckfiltrieren und Waschen.

5. Verfahren nach Anspruch 1 oder 2, wobei die feste Zusammensetzung erhalten wird aus der zweiten vorbehandelten lignozellulosischen Biomasse durch ein Verfahren, umfassend die Schritte von:
a) Hydrolysieren mindestens einer Fraktion der zweiten vorbehandelten lignozellulosischen Biomasse in der Gegenwart eines Hydrolysekatalysators, um eine hydrolysierte Zusammensetzung zu erhalten, die Komplex-Zucker und Lignin und die Lösung von Wasser und löslichen Zuckern umfasst;
b) Umwandlung mindestens einer Fraktion der löslichen Zucker in der hydrolysierten Zusammensetzung, um eine umgewandelte Zusammensetzung zu erhalten, die mindestens ein zuckerabgeleitetes Produkt umfasst, und die feste Zusammensetzung;
c) Entfernen einer Fraktion des mindestens einen zuckerabgeleiteten Produkts.

6. Verfahren nach Anspruch 5, wobei die Entfernung der Fraktion des mindestens einen zuckerabgeleiteten Produkts durchgeführt wird mittels eines Verfahrens ausgewählt aus der Gruppe, bestehend aus Dekantieren, Sedimentieren, Zentrifugieren, Filtrieren, Druckfiltrieren, Waschen, Verdampfen und Destillieren.

7. Verfahren nach irgendeinem der Ansprüche 3 bis 6, wobei der Hydrolysekatalysator ein zweites Enzym oder Enzymmischung umfasst.

8. Verfahren nach irgendeinem der Ansprüche 3 bis 6, wobei der Hydrolysekatalysator ausgewählt ist aus der Gruppe, bestehend aus Wasserstoffionen, organischen Säuren und anorganischen Säuren.

9. Verfahren nach irgendeinem der Ansprüche 5 bis 8, wobei die Umwandlung der hydrolysierten Zusammensetzung eine Fermentierung umfasst.

10. Verfahren nach Anspruch 9, wobei das mindestens eine zuckerabgeleitete Produkt Ethanol umfasst.

11. Verfahren nach irgendeinem der Ansprüche 2 bis 10, wobei der Teil der Kultivierungszeit unter einfachen zuckerabgereicherten Bedingungen mindestens 75% der Kultivierungszeit ist.

12. Verfahren nach irgendeinem der Ansprüche 2 bis 10, wobei der Teil der Kultivierungszeit unter zuckerabgereicherten Bedingungen die gleiche ist wie die Kultivierungszeit.

13. Verfahren nach irgendeinem der Ansprüche 2 bis 12, wobei die Menge an zugesetztem Zucker oder Zuckern in einem Bereich zwischen 0 und 5 Gew.-% der zuckerabgereicherten Kultivierungsumgebung ist, auf Trockenbasis.

14. Verfahren nach irgendeinem der Ansprüche 2 bis 12, wobei in der zuckerabgereicherten Kultivierungsumgebung kein einfacher Zucker zugegeben ist.

15. Verfahren nach irgendeinem der Ansprüche 1 bis 14, wobei mindestens das erste Enzym geerntet wird durch Trennung der zuckerabgereicherten Kultivierungsumgebung in mindestens eine geerntete Zusammensetzung, umfassend mindestens eine Fraktion der Menge des ersten Enzyms, und eine erschöpfte Zusammensetzung, umfassend mindestens eine Fraktion einer Menge des festen Rückstands der festen Zusammensetzung.

16. Verfahren nach Anspruch 15, wobei die Gesamtmenge der Komplex-Zucker in der erschöpften Zusammensetzung in einem Bereich von 0 bis 30 Gew.-%, auf Trockenbasis, vorliegt.

17. Verfahren nach irgendeinem der Ansprüche 1 bis 16, wobei das erste Enzym weiterhin verwendet wird, um die erste lignozellulosische Biomasse zu hydrolysieren, und die erste lignozellulosische Biomasse und die zweite vorbehandelte lignozellulosische Biomasse umfassen beide lignozellulosische Biomasse, abgeleitet aus der Gruppe bestehend aus demselben Gras-Genus und derselben Gras-Spezies.

## Revendications

1. Procédé en vue de produire au moins une première enzyme apte à hydrolyser une première biomasse lignocellulosique prétraitée, lequel procédé comporte l'étape consistant à cultiver la cellule hôte dans un milieu de culture appauvri en sucre, qui contient la cellule hôte, de l'eau et une composition solide, elle-même préparée à partir d'une deuxième biomasse lignocellulosique prétraitée contenant un sucre complexe, choisi parmi des xylanes et glucanes polymères insolubles dans l'eau, et une lignine, étant entendu que le rapport de la quantité totale de sucres complexes à la quantité totale de lignine dans la composition solide est inférieur au rapport de la quantité totale de sucres complexes à la quantité totale de lignine dans la deuxième biomasse lignocellulosique prétraitée, et étant entendu que ladite composition solide est préparée par élimination d'une fraction, et non de la totalité, des sucres complexes contenus dans la deuxième biomasse lignocellulosique prétraitée.

2. Procédé selon la revendication 1, dans lequel on effectue la culture dans des conditions d'appauvrissement en sucres simples, en travaillant avec une proportion de sucre(s) simple(s) ajouté(s), rapportée au poids du milieu de culture appauvri en sucre et calculée sur matière sèche, comprise dans l'intervalle allant de 0 % à 10 % en poids pendant une partie de la durée de culture représentant au moins 50 % de la durée de culture, étant entendu que lesdits sucres simples sont choisis parmi des sucres monomères.

3. Procédé selon la revendication 1 ou la revendication 2, dans lequel on prépare la composition solide à partir de la deuxième biomasse lignocellulosique prétraitée, au moyen d'un procédé comportant les étapes suivantes :
(a) hydrolyser au moins une fraction de la deuxième biomasse lignocellulosique prétraitée, en présence d'un catalyseur d'hydrolyse, de manière à produire une composition hydrolysée contenant une composition solide et une solution constituée d'eau et de sucres solubles,
(b) et éliminer de la composition hydrolysée une fraction représentant au moins 50 % de la solution constituée d'eau et de sucres solubles.

4. Procédé selon la revendication 3, dans lequel on effectue l'élimination de la solution constituée d'eau et de sucres solubles à l'aide d'au moins une technique choisie parmi la décantation, la sédimentation, la centrifugation, la filtration, la filtration sur filtre-presse et le lavage.

5. Procédé selon la revendication 1 ou la revendication 2, dans lequel on prépare la composition solide à partir de la deuxième biomasse lignocellulosique prétraitée, au moyen d'un procédé comportant les étapes suivantes :
(a) hydrolyser au moins une fraction de la deuxième biomasse lignocellulosique prétraitée, en présence d'un catalyseur d'hydrolyse, de manière à produire une composition hydrolysée contenant lesdits sucres complexes et ladite lignine, et une solution constituée d'eau et de sucres solubles,
(b) transformer au moins une fraction des sucres solubles de la composition hydrolysée, de manière à produire une composition transformée contenant au moins un produit issu du sucre et la composition solide,
(c) éliminer une fraction du produit issu du sucre au nombre d'au moins un.

6. Procédé selon la revendication 5, dans lequel on effectue l'élimination du produit issu du sucre, au nombre d'au moins un, à l'aide d'au moins une technique choisie parmi la décantation, la sédimentation, la centrifugation, la filtration, la filtration sur filtre-presse, le lavage, l'évaporation et la distillation.

7. Procédé selon n'importe lesquelles des revendications 3 à 6, dans lequel le catalyseur d'hydrolyse comprend une deuxième enzyme ou un mélange d'enzymes.

8. Procédé selon n'importe lesquelles des revendications 3 à 6, dans lequel le catalyseur d'hydrolyse est choisi dans l'ensemble constitué par des ions hydrogène, des acides organiques et des acides minéraux.

9. Procédé selon n'importe lesquelles des revendications 5 à 8, dans lequel la transformation de la composition hydrolysée comporte une fermentation.

10. Procédé selon la revendication 9, dans lequel le produit issu du sucre, au nombre d'au moins un, comprend de l'éthanol.

11. Procédé selon n'importe lesquelles des revendications 2 à 10, dans lequel la partie de la durée de culture pratiquée dans des conditions d'appauvrissement en sucres simples représente au moins 75 % de la durée de culture.

12. Procédé selon n'importe lesquelles des revendications 2 à 10, dans lequel la partie de la durée de culture pratiquée dans des conditions d'appauvrissement en sucre est égale à la durée de culture.

13. Procédé selon n'importe lesquelles des revendications 2 à 12, dans lequel la proportion de sucre(s) simple(s) ajouté(s), rapportée au poids du milieu de culture appauvri en sucre et calculée sur matière sèche, est comprise dans l'intervalle allant de 0 % à 5 % en poids.

14. Procédé selon n'importe lesquelles des revendications 2 à 12, dans lequel il n'y a pas de sucre simple ajouté dans le milieu de culture appauvri en sucre.

15. Procédé selon n'importe lesquelles des revendications 1 à 14, dans lequel on récolte au moins la première enzyme en séparant le milieu de culture appauvri en sucre en au moins une composition de récolte, contenant au moins une fraction de la première enzyme, et une composition épuisée, contenant au moins une fraction du résidu solide de la composition solide.

16. Procédé selon la revendication 15, dans lequel la proportion totale desdits sucres complexes dans la composition épuisée, calculée sur matière sèche, est comprise dans l'intervalle allant de 0 % à 30 % en poids.

17. Procédé selon n'importe lesquelles des revendications 1 à 16, dans lequel on utilise par ailleurs la première enzyme pour hydrolyser la première biomasse lignocellulosique, et la première biomasse lignocellulosique et la deuxième biomasse lignocellulosique prétraitée comprennent toutes les deux de la biomasse lignocellulosique provenant du groupe constitué par le même genre de graminées et la même espèce de graminées.
